# EUROPEAN PATENT APPLICATION

(11) **EP 4 512 457 A1**
(43) Date of publication of application: **26.02.2025**
(21) Application number: 24192276.4
(22) Date of filing: 01.08.2024
(51) Int. Cl.: A61M 39/00, A61M 39/26, A61M 39/10

(54) **EASY TO DISENGAGE SEPARATOR**

(30) Priority: 21.08.2023 TW 112131348; 30.05.2024 TW 113120103
(71) Applicant: Tsai, Hsi-Chin, 238 New Taipei City (TW)
(72) Inventor: Tsai, Hsi-Chin, 238 New Taipei City (TW); Chou, Chih-Hsuan, 220 New Taipei City (TW); Su, Chun-Yi, 220 New Taipei City (TW)
(74) Representative: Gee, Steven William

(57) **Abstract**

Provided is an easy to disengage separator, including: a valve body, a screw-coupling structure, an output end, and a snap-on needleless connector module. The snap-on needleless connector module includes a movable closed connector and a needleless connector. The movable closed connector includes a main body, a first coupling part and a first elastic valve. The main body includes a sleeve and a first engaging part. The first engaging part is a bump and is provided on the sleeve. The needleless connector includes a shell, a second coupling part and a second elastic valve. The shell or the second coupling part has a second engaging part, the second engaging part is a bump, and the force of the first engaging part fixed on the second engaging part is 1.4-8.1 Newton-meters, easy to disengage. Thus, the movable closed connector and the needleless connector will immediately disengage to prevent leakage and damages.

## Description

### CROSS-REFERENCE TO RELATED APPLICATION

This application claims the priorities of Taiwanese patent application No. 112131348, filed on August 21, 2023, and Taiwanese patent application No. 113120103, filed on May 30, 2024, which are incorporated herewith by reference.

### BACKGROUND OF THE INVENTION

### 1. Field of the Invention

The present invention relates generally to a separator for an infusion system, and more particularly, to an easy to disengage separator.

### 2. The Prior Arts

US Patent No. 10,688,295B2 disclosed a liquid transfer device for use with infusion liquid container, and the medicine mixing procedure includes the following steps:

According to the doctor's prescription, a vial bottle containing a certain powder additive is prepared; according to the doctor's prescription, an infusion liquid bag containing a diluting solution is prepared; the vial bottle is sheathed on the integral vial adapter of the needleless additive port, the puncturing member of the vial adapter punctures the IV spike of the vial bottle; one end of the puncturing member punctures the film of the infusion tube of the infusion liquid bag; the infusion liquid bag is on the top and the vial bottle is on the bottom. When the infusion liquid bag is squeezed, the diluting solution in the liquid infusion bag passes through the opening and channel of the puncturing part, the second flow channel of the output part, the first flow channel of the needleless additive port, and the puncturing member to enter the inside of the vial bottle; shake the vial bottle to slightly mix the powder in the vial bottle to obtain a high-concentration medicine; then put the liquid infusion bag at the bottom and the vial bottle at the top; squeeze the liquid infusion bag to release the air in the liquid infusion bag to pass through the through hole and long hole of the puncturing part, the second flow channel of the output part, the first flow channel of the needleless additive port and puncturing member to enter the inside of the vial bottle and release the squeezing hand to suck out the liquid inside the vial bottle, and repeat the operation until the liquid in the vial bottle is completely absorbed into the liquid infusion bag.

The infusion procedure for the conventional mixing device includes the following steps: step (a), remove the plug body of the administration port from the other end of the tube body; step (b), arrange the infusion drip set at the other end of the tube body and penetrate the sealing membrane, so that the infusion drip set can communicate with the second flow channel of the output part through the third flow channel of the tube body and; step (c), place the liquid infusion bag at the top and the infusion drip set at the bottom so that the diluted medicine inside the liquid infusion bag passes through the through hole and long hole of the puncturing part, the second flow channel of the output part, the third flow channel of the tube body, and the infusion drip set in sequence to enter the patient's body.

However, when the high-concentration medicinal liquid in the vial bottle passes through the long hole of the puncturing part, due to the entry of air and the inability to absorb all the medicinal liquid at one time during suction, some high-concentration medicinal liquid will form residue attached to the long hole of the puncturing part. When the air in the liquid infusion bag passes through the long hole of the puncturing part, the air will push all the high-concentration medicinal liquid attached to the inner wall of the channel of the puncturing part to the space between the second flow channel and the insertion hole of the output part, and blocked by the tube body, forming residual high-concentration medicinal liquid. Even if the liquid infusion bag is loosened, the high-concentration medicinal liquid remaining in the space between the second flow channel of the output part and the insertion hole cannot be pushed into the liquid infusion bag by the flow of air. Therefore, when the chemical mixing process is repeated, the space between the second flow channel of the output part and the insertion hole will be filled with residual high-concentration medicinal liquid due to air squeezing.

Because the high-concentration medicinal liquid remaining in the space between the second flow channel of the output part and the insertion hole has not been diluted, the concentration is too high. Once the nursing staff uses the conventional medicine mixing device to perform the infusion procedure, the high-concentration medicinal liquid remaining in the space between the second flow channel of the output part and the insertion hole will first enter the patient's body, and the diluted medicinal liquid in the liquid infusion bag will then enters the patient's body. However, the entry of the residual high-concentration medicinal liquid into the patient's body will cause adverse reactions in the patient's body. Therefore, patients often report feeling very uncomfortable in the early stages of the infusion procedure. If this conventional medicine mixing device is used to infuse medicines into patients who require precise and quantitative administration, such as pregnant women or critically ill patients, serious consequences may occur, and even life-threatening consequences may occur.

Some nursing staff have noticed the above situation and will remove the residual in the space between the second flow channel of the output part and the insertion hole after step (a) of the infusion procedure and before step (b). High-concentration medicinal liquid is discharged from the third flow channel of the tube body. However, this practice directly leads to a reduction in the dose infused into the patient's body, which is inconsistent with the doctor's prescriptions, thereby reducing the therapeutic effect and resulting in medicine waste.

Furthermore, in step (c) of the infusion procedure of the conventional medicine mixing device, because the first flow channel of the needleless additive port and the second flow channel of the output part are still connected, part of the diluted medicine will flow from the second flow channel of the output part to enter the inside of the vial bottle through the first flow channel of the needleless additive port and the puncturing member, resulting in a reduction in the dose infused into the patient's body, which is inconsistent with the doctor's prescriptions, thereby reducing the therapeutic effect and resulting in medicine waste.

Moreover, because the needleless additive port has no sealing effect, the vial bottle cannot be removed from the needleless additive port, otherwise the residual high-concentration medicinal liquid will leak out through the needleless additive port. Therefore, the conventional medicine mixing device can only be used once and cannot be reused.

Furthermore, the conventional infusion system includes a medicine mixing device, an infusion drip set, a dispenser, a separator, and an retention needle. Because certain patients tend to move agitatedly, the dispenser and separator are easily separated, and the retention needle is easily separated from the artificial blood vessel, causing the medical solution and blood to leak out.

U.S. Patent No. 6,651,956B2 disclosed a slit-type swabable valve, U.S. Patent No. 9,415,199B2 disclosed a leak proof needleless medical connector, and U.S. Patent No. 10,201,693B2 disclosed a closed male luer. The above three patents can be disposed at the needleless additive port of the conventional needleless control valve shown in FIG. 1 of US10,688,295B2 to be used for connecting the infusion drip set. The infusion drip set can deliver concentrated medicinal liquid into the liquid infusion bag through the above-mentioned patented combination medicine mixing device. The slit-type swabable valve has a sealing effect and solves the problem that the needleless additive port of US 10,688,295B2 has no sealing effect. Therefore, the medicine mixing device of the above patent combination can be reused. Unfortunately, the above patent combination cannot solve other problems of US10,688,295B2.

United States Patent No. 11235135B2 discloses a needleless infusion closed connector to prevent leakage of medicinal liquid, which has a first connecting component and a second connecting component. The first connecting component has a first sleeve and a first elastic valve. The first sleeve forms a first opening, and the first elastic valve tends to seal the first opening. The second connection component has a second sleeve and a second elastic valve. The second sleeve forms a second opening, and the second elastic valve tends to seal the second opening. The first elastic valve tends to seal the first opening of the first sleeve, and the second elastic valve tends to seal the second opening, thereby preventing the syringe and the infusion catheter connecting assembly from leakage of the medicine liquid remained in the medicine bottle and human body end connecting assembly. It can prevent leakage of medicinal solution and can be bidirectionally sealed to prevent leakage when connecting and removing after adding medication or infusion.

### SUMMARY OF THE INVENTION

A primary objective of the present invention is to provide an easy to disengage separator. Even if the patient moves erratically, the movable closed connector and the needleless connector will immediately separate and prevent the leakage of medicinal liquid and blood, and alcohol can be used with cotton pads to sterilize for reuse without the need to replace.

In order to achieve the aforementioned objective, the present invention provides an easy to disengage separator, including a valve body, a screw-coupling structure, an output end, and a snap-on needleless connector module. An internal thread at a first end of the screw-coupling structure is threaded with an external thread at a first end of the valve body and defines an input end of the separator. The screw-coupling structure has an inner conduit. A first end of the output end is disposed on a second end of the valve body. The snap-on needleless connector module includes a movable closed connector and a needleless connector. The movable closed connector includes a main body, a first coupling part and a first elastic valve. The main body includes a sleeve, a conduit, a positioning part and a first engaging part. The conduit is arranged inside the sleeve and includes a first channel and a first opening, the positioning part is located inside the sleeve and is provided on the outside of the conduit, the first engaging part is a bump and is provided on the sleeve, and the first coupling part is provided on the sleeve. The first elastic valve includes a first circumferential part and a first sealing part. Two ends of the first circumferential part are respectively provided on the positioning part and the first coupling part, the first sealing part is located in the first channel, a first end of the first sealing part is connected to an inner side of the first circumferential part and a plurality of through holes are provided, the through holes communicate with the first channel, and a second end of the first sealing part closes the first opening. The needleless connector includes a shell, a second coupling part and a second elastic valve. The shell or the second coupling part has a second engaging part, the second engaging part is a bump, and is provided on an outer side of the shell or an outer side of the second coupling part, a first end of the shell has a second opening, and the second coupling part is provided on a second end of the shell, includes a third opening, and is combined with the screw-coupling structure; and the third opening communicates with the inner conduit. The second elastic valve includes a second circumferential part and a second sealing part. The second circumferential part is provided inside the shell and the second coupling part and has a second channel, and the second channel communicates with the third opening. The third opening communicates with the inner conduit. The second sealing part is provided inside the shell and has a slit, and the second sealing part closes the second opening. Wherein, when the movable closed connector is docked with the needleless connector, the first engaging part is fixed on the second engaging part, and the force of the first engaging part being fixed on the second engaging part is 1.4-8.1 Newton-meters, the conduit compresses the second sealing part, the second sealing part compresses the second circumferential part, so that the second sealing part is away from the second opening, and the conduit passes through the slit of the second sealing part and enter the inside of the second sealing part, the first end of the shell is against an inner wall of the sleeve, the positioning part moves along the conduit in a direction away from the needleless connector and compresses the first circumferential part, so that the second end of the first sealing part is separated from the first opening, and the first channel and the second channel become communicated with the inner conduit. Wherein, when the movable closed connector is separated from the needleless connector, the first engaging part is disengaged from the second engaging part, and the conduit is separated from the second sealing part, so that the slit of the second sealing part becomes closely fitting, the second sealing part is restored by the elastic force of the second circumferential part and closes the second opening, the first end of the shell is separated from the sleeve, and the positioning part and the first sealing part are reset by the elastic reset of the first circumferential part to cause the second end of the first sealing part to close the first opening.

In order to achieve the aforementioned objective, the present invention provides an easy to disengage separator, including a valve body, a screw-coupling structure, an output end, and a snap-on needleless connector module. An internal thread at a first end of the screw-coupling structure is threaded with an external thread at a first end of the valve body and defines an input end of the separator. The screw-coupling structure has an inner conduit. A first end of the output end is disposed on a second end of the valve body. The snap-on needleless connector module includes a movable closed connector and a needleless connector. The movable closed connector includes a main body, a first coupling part and a first elastic valve. The main body includes a sleeve, a conduit, a positioning part and a first engaging part. The conduit is arranged inside the sleeve and includes a first channel and a first opening, the positioning part is located inside the sleeve and is provided on the outside of the conduit, the first engaging part is a bump and is provided on the sleeve, and the first coupling part is provided on the sleeve. The first elastic valve includes a first circumferential part and a first sealing part. One end of the first circumferential part is provided on the positioning part and the first coupling part, the first sealing part is located in the first channel, a first end of the first sealing part is connected to an inner side of the first circumferential part and a plurality of through holes are provided, the through holes communicate with the first channel, and a second end of the first sealing part closes the first opening. The needleless connector includes a shell, a second coupling part, a communication tube, a third elastic valve, and a fourth elastic valve. The shell has a second engaging part, and the second engaging part is a bump is provided on an outer side of the shell or an outer side of the second coupling part. A first end of the shell has a second opening, and the second coupling part is provided on a second end of the shell, has a third opening, and is integrally formed with the screw-coupling structure. The third opening communicates with the inner conduit. The communication tube is provided in the shell and includes an upper cylinder, a large-diameter part and a lower cylinder. The upper cylinder is arranged above the large-diameter part with a top as a closed end, and at least one through hole is provided. The at least one through hole penetrates one side wall of the upper cylinder and communicates with the inside of the upper cylinder. The lower cylinder is arranged below the large-diameter part with a bottom end as an open end, and the inside of the upper cylinder, the inside of the large-diameter part, and the inside of the lower cylinder communicate with one another; the third elastic valve is arranged in the shell and has a third circumferential part and a third sealing part. The third circumferential part is arranged around the outside of the upper cylinder and its bottom end is against the large-diameter part, the third sealing part is provided at the top of the third circumferential part and has a slit; the fourth elastic valve is provided in the shell and has a fourth circumferential part and a fourth sealing part, the fourth circumferential part is arranged around the outside of the lower cylinder and its top end is against the large-diameter part, and the fourth sealing part is arranged at the bottom end of the fourth circumferential part and is against an inner wall of the second coupling part. A second end of the shell presses the top of the fourth sealing part, the fourth sealing part has a slit, and the slit of the fourth sealing part is aligned with the inside of the lower cylinder and the third opening. Wherein, when the movable closed connector is docked with the needleless connector, the first engaging part is fixed on the second engaging part, and the force of the first engaging part being fixed on the second engaging part is 1.4-8.1 Newton-meters, the upper cylinder pushes open the slit of the third sealing part, the upper cylinder pushes against and compresses the first sealing part, and the upper cylinder passes through the first opening and enters the inside of the conduit, the conduit compresses the third sealing part, the third sealing part compresses the third circumferential part, so that the at least one through hole of the upper cylinder is exposed outside the third sealing part and is located inside the conduit, the third circumferential part pushes against the large-diameter part, the large-diameter part compresses the fourth circumferential part, and the lower cylinder pushes open the slit of the fourth sealing part and enters the third opening, so that the first channel, the communication tube, and the inner conduit are connected. Wherein, when the movable closed connector is separated from the needleless connector, the first engaging part is disengaged from the second engaging part, and the fourth circumferential part is pushed against the large-diameter part by its elastic force, so that the communication tube and the third elastic valve move, and the lower cylinder returns to the inside of the fourth circumferential part to make the slit of the fourth sealing part tightly closed; the third circumferential part is pushed against the conduit by its elastic force to drive the movable closed connector to move, causing the upper cylinder to break away from the conduit and return to the inside of the third circumferential part. The conduit breaks away from the third sealing part, causing the slit of the third sealing part to be tightly closed. The third sealing part is restored by the elastic force of the third circumferential part and closes the second opening, the first end of the shell is disengaged from the sleeve, and the first sealing part is reset by its elastic force, so that the second end of the first sealing part closes the first opening.

The effect of the present invention is that the force of fixing the first engaging part to the second engaging part is 1.4-8.1 Newton-meters (i.e., 1 to 6 pounds per foot), which is easy to separate. Even if the patient moves erratically, the movable closed connector and the needleless connector will immediately separate to prevent leakage of medicine and blood, and can be disinfected with alcohol swabs for reuse without replacement.

### BRIEF DESCRIPTION OF THE DRAWINGS

The present invention will be apparent to those skilled in the art by reading the following detailed description of a preferred embodiment thereof, with reference to the attached drawings, in which:
FIG. 1 is a schematic diagram of the infusion system of the present invention.
FIG. 2 is an exploded cross-sectional view of the first embodiment of the second medicine mixing device of the present invention.
FIG. 3 is an exploded cross-sectional view of the second embodiment of the second medicine mixing device of the present invention.
FIG. 4 is an exploded cross-sectional view of the separator of the present invention.
FIG. 5A is a schematic diagram of the first embodiment of the second medicine mixing device of the present invention performing medicine mixing and infusion.
FIG. 5B is a schematic diagram of area A of FIG. 5A.
FIG. 6 is a schematic diagram of the second embodiment of the second medicine mixing device of the present invention performing mixing medicine and infusion.
FIG. 7A is a schematic diagram of the infusion of the separator of the present invention.
FIG. 7B is a schematic diagram of area B of FIG. 7A.
FIG. 8 is a schematic diagram of another embodiment of the first closed needleless connector module of the present invention.
FIG. 9A is an exploded perspective view of the second embodiment of the third closed needleless connector module of the present invention.
FIG. 9B is an exploded cross-sectional view of the second embodiment of the third closed needleless connector module of the present invention.
FIG. 9C is a cross-sectional view of the second embodiment of the third closed needleless connector module of the present invention.
FIG. 9D is a schematic diagram of area C of FIG. 9C.
FIG. 9E is a perspective cross-sectional view of area C in FIG. 9C.
FIG. 10A is an exploded perspective view of the third embodiment of the third closed needleless connector module of the present invention.
FIG. 10B is an exploded cross-sectional view of the third embodiment of the third closed needleless connector module of the present invention.
FIG. 10C is a cross-sectional view of the third embodiment of the third closed needleless connector module of the present invention.
FIG. 10D is a schematic diagram of area D of FIG. 10C.
FIG. 11A is a perspective view of the syringe and valve body in other uses.
FIG. 11B is a side view of the syringe and valve body in other uses.
FIG. 12A is a cross-sectional view of the syringe and the movable closed connector integrally formed.
FIG. 12B is a cross-sectional view of a syringe and another movable closed connector integrally formed.
FIG. 13A is a perspective view of the fourth embodiment of the third closed needleless connector module of the present invention.
FIG. 13B is an exploded perspective view of the fourth embodiment of the third closed needleless connector module of the present invention.
FIG. 13C is a cross-sectional view of the fourth embodiment of the third closed needleless connector module of the present invention.
FIG. 13D is a cross-sectional view of the fourth embodiment of the third closed needleless connector module of the present invention in other uses and with the syringe providing positive pressure.
FIG. 13E is a schematic diagram of area E of FIG. 13D.
FIG. 13F is a schematic diagram of the continuous actions of the balance pressure valve of the fourth embodiment of the third closed needleless connector module of the present invention being deformed under the influence of positive pressure.
FIG. 13G is a cross-sectional view of the fourth embodiment of the third closed needleless connector module of the present invention for other uses and the balanced pressure valve provides a backflow prevention function.
FIG. 13H is a schematic diagram of area F of FIG. 13G.
FIG. 13I is a cross-sectional view of the fourth embodiment of the third closed needleless connector module of the present invention in other uses and when the balance pressure valve loses the backflow prevention function.
FIG. 13J is a schematic diagram of area G of FIG. 13I.
FIG. 14A is a perspective view of the fifth embodiment of the third closed needleless connector module of the present invention.
FIG. 14B is an exploded perspective view of the fifth embodiment of the third closed needleless connector module of the present invention.
FIG. 14C is a cross-sectional view of the fifth embodiment of the third closed needleless connector module of the present invention.
FIG. 14D is a cross-sectional view of the fifth embodiment of the third closed needleless connector module of the present invention in other uses and with the syringe providing positive pressure.
FIG. 14E is a schematic diagram of area H of FIG. 14D.
FIG. 14F is a cross-sectional view of the fifth embodiment of the third closed needleless connector module of the present invention in other uses and with the balance pressure valve providing a backflow prevention function.
FIG. 14G is a schematic diagram of area I of FIG. 14F.
FIG. 15A is a perspective view of the sixth embodiment of the third closed needleless connector module of the present invention.
FIG. 15B is an exploded perspective view of the sixth embodiment of the third closed needleless connector module of the present invention.
FIG. 15C is a cross-sectional view of the sixth embodiment of the third closed needleless connector module of the present invention.
FIG. 16A is an exploded cross-sectional view of the seventh embodiment of the third closed needleless connector module of the present invention.
FIG. 16B is a cross-sectional view of the seventh embodiment of the third closed needleless connector module of the present invention.

### DETAILED DESCRIPTION OF THE PREFERRED EMBODIMENT

The accompanying drawings are included to provide a further understanding of the invention, and are incorporated in and constitute a part of this specification. The drawings illustrate embodiments of the invention and, together with the description, serve to explain the principles of the invention.

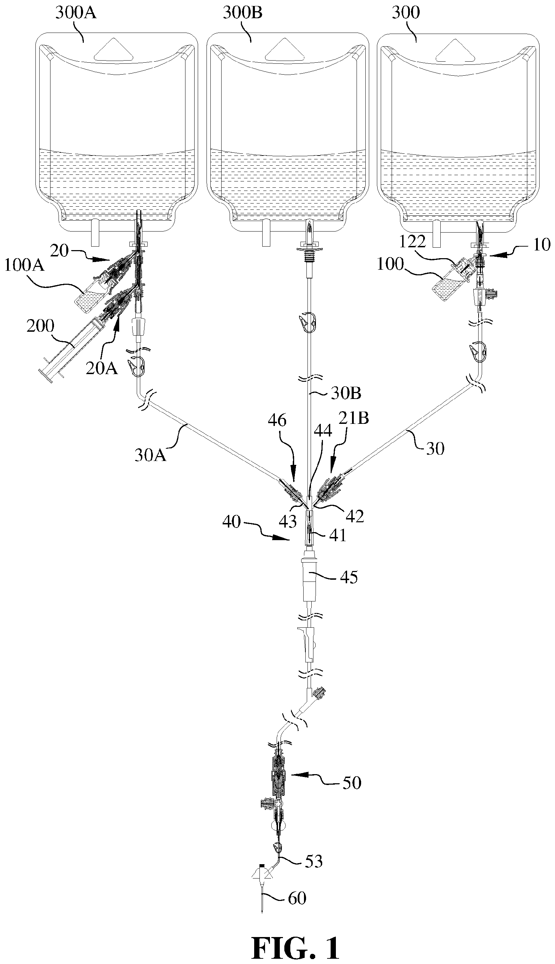

As shown in FIG. 1, the present invention provides an infusion system, including a first mixing device 10, two second mixing devices 20, 20A, three infusion drip sets 30, 30A, 30B, a dispenser 40, a separator 50, and a retention needle 60.

The first medicine mixing device 10 includes a puncturing part 11, a needleless additive port 12, an output part 13, and a plug member 14. The following uses the second medicine mixing device as an example to describe the details of the aforementioned structure.

A first end of the puncturing part 11 is a sharp end, and a U-shaped through hole 111 and three side holes 112 are disposed on one side wall of the puncturing part 11. The internal space of the puncturing part 11 is divided into a first long hole 114 and three second long holes 115 by a first partition 113. The first long hole 114 communicates with the U-shaped through hole 111, and each second long hole 115 communicates with each side hole 112. In addition, a distance D1 from the top of the U-shaped through hole 111 to the sharp end of the puncturing part 11 is less than a distance D2 from the top of the side hole 112 to the sharp end of the puncturing part 11. In other words, the top of the U-shaped through hole 111 is closer to the sharp end of the puncturing part 11 and forms a U-shaped hole, and the top of the side hole 112 is farther from the sharp end of the puncturing part 11.

The needleless additive port 12 includes a coupling seat 121. The coupling seat 121 is integrally formed on one side of a second end of the puncturing part 11 and defines a first flow channel 1211, and the first flow channel 1211 communicates with the U-shaped through hole 111.

The output part 13 is disposed at the second end of the puncturing part 11, and has a second flow channel 131 and a insertion hole 132. The second flow channel 131 is connected between the second long holes 115 and the insertion hole 132. In the present embodiment, the outer surface of the output part 13 is a smooth curved surface.

The first flow channel 1211 and the second flow channel 131 are separated by a second partition 15 between the needleless additive port 12 and the output part 13, and the first partition 113 is connected to the second partition 15.

The plug member 14 has a tube body 141. The tube body 141 defines a third flow channel 1411, and a first end of the tube body 141 is inserted into the insertion hole 132, so that the second flow channel 131 communicates with the third flow channel 1411. A blocking part 1412 is disposed inside the third flow channel 1411 to close the third flow channel 1411.

As shown in FIG. 1, the needleless additive port 12 of the first medicine mixing device further includes an integral vial adaptor122. The integral vial adaptor122 is integrally formed on the coupling seat 121, has a puncturing member 1221, and is used to be combined with a vial bottle 100.

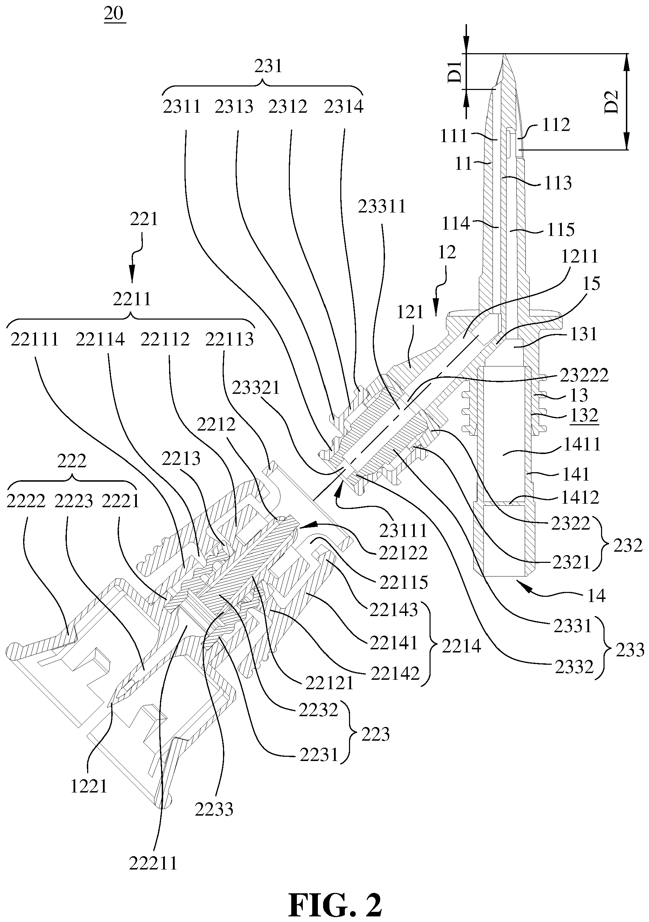

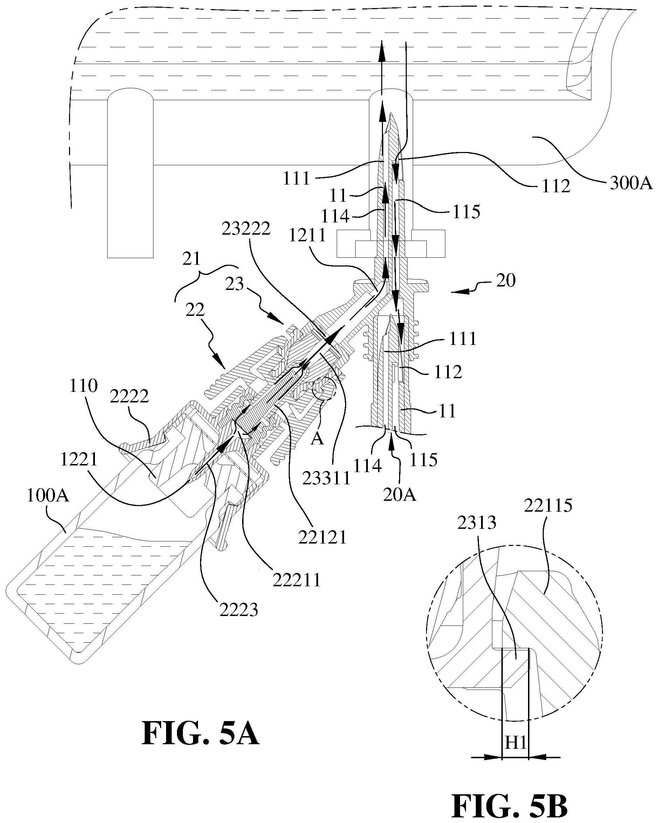

As shown in FIGS. 2 and 5A, the structural difference between the second medicine mixing device 20 and the first medicine mixing device 10 is that the second medicine mixing device 20 further includes a first closed needleless connector module 21. The first closed needleless connector module 21 replaces the integral vial adaptor 122. Furthermore, the first closed needleless connector module 21 includes a movable closed connector 22 and a first needleless connector 23.

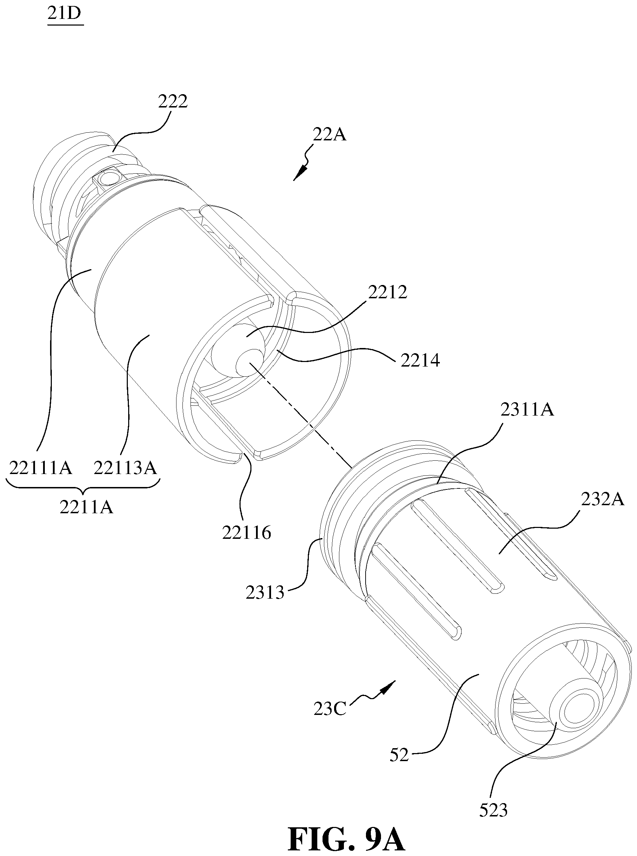

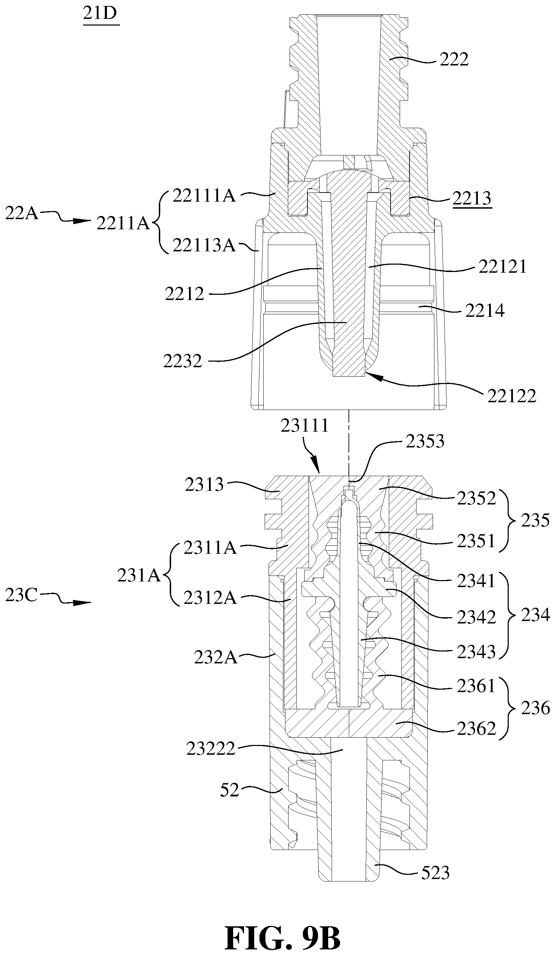

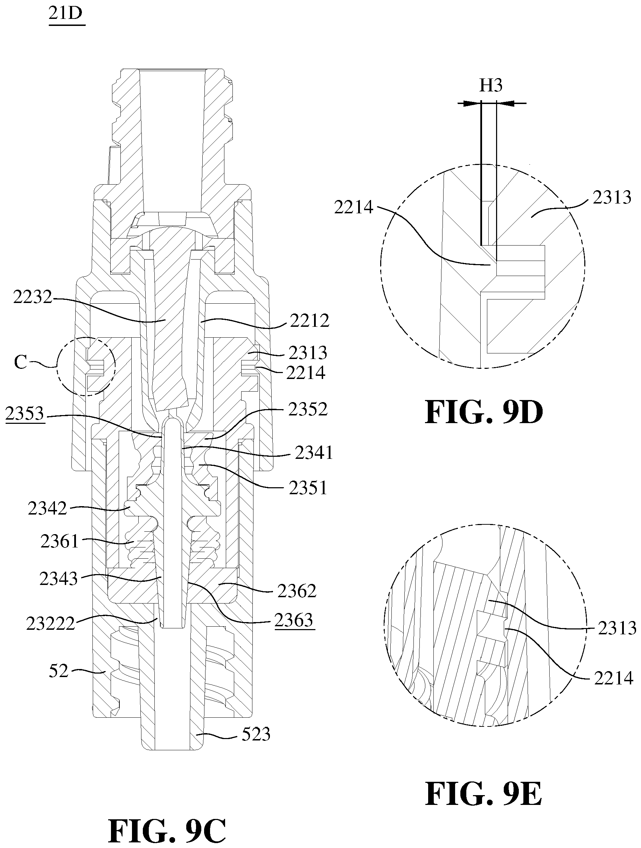

The movable closed connector 22 includes a body 221, a first coupling part 222, and a first elastic valve 223. The body 221 includes a sleeve 2211, a conduit 2212, a positioning part 2213 and a first engaging part 2214. The sleeve 2211 includes an upper tube part 22111, a middle cylinder part 22112 and a lower tube part 22113. The middle cylinder part 22112 is disposed between a first end of the upper tube part 22111 and a first end of the lower tube part 22113. A first through hole 22114 is disposed between one side wall of the upper tube part 22111 and a side wall of the middle cylinder part 22112, and a second through hole is disposed between the side wall of the middle cylinder part 22112 and one side wall of the lower tube part 22113. 22115. The conduit 2212 extends from the inside of the upper tube part 22111 to the inside of the middle cylinder part 22112 and has a first channel 22121 and a first opening 22122. The positioning part 2213 is located inside the upper tube part 22111 and is disposed outside the conduit 2212. The first engaging part 2214 includes an arm part 22141, a connecting part 22142 and a hook part 22143. The connecting part 22142 is disposed at the upper tube part 22111, passes through the first through hole 22114 from the inside of the upper tube part 22111, and connects the arm part 22141; and the hook part 22143 is disposed at one end of the arm part 22141 and located in the second through hole 22115. The first coupling part 222 includes a first end part 2221, a second end part 2222 and a connecting channel 2223. The first end part 2221 is disposed at a second end of the upper tube part 22111 and has a groove 22211, and a groove wall of the groove 22211 is arc-shaped. The second end 2222 is an integral vial adaptor, has a puncturing member 1221, and is used to be combined with a vial bottle 100A (see FIGS. 1 and 9); and the connecting channel 2223 runs through the first end 2221 and the second end 2222 and communicates with the groove 22211. The first elastic valve 223 includes a first circumferential part 2231 and a first sealing part 2232. Two ends of the first circumferential part 2231 are respectively disposed at the positioning part 2213 and the first end part 2221. The first sealing part 2232 is located in the channel 22121. A first end of the first sealing part 2232 is connected to an inner side of the first circumferential part 2231 and disposed with a plurality of through holes 2233. The through holes 2233 are connected with the first channel 22121, the through holes 2233 are connected with the grooves 22211, and a second end of the first sealing part 2232 closes the first opening 22122.

The first needleless connector 23 includes a shell 231, a second coupling part 232, and a second elastic valve 233. The shell 231 includes an upper shell 2311, a lower shell 2312, a second engaging part 2313, and a stabilizing structure 2314. A first end of the upper shell 2311 has a second opening 23111. An outer diameter of the upper shell 2311 is smaller than that of the lower shell 2311. The second engaging part 2313 is a bump and is disposed on an outer side of a first end of the lower shell 2312. The stabilizing structure 2314 is a bump and is disposed on an outer side of a second end of the lower shell 2312. The second coupling part 232 includes a first end part 2321 and a second end part 2322. The first end part 2321 is disposed at a second end of the lower shell 2312. The second end part 2322 has a third opening 23222 and is used to combine with the coupling seat 121. The second elastic valve 233 includes a second circumferential part 2331 and a second sealing part 2332. The second circumferential part 2331 is disposed inside the lower shell 2312 and the second coupling part 232 and has a second channel 23311. The second channel 23311 communicates with the third opening 23222. The second sealing part 2332 is disposed inside the upper shell 2311 and has a slit 23321. The second sealing part 2332 closes the second opening 23111.

As shown in FIG. 5A, and refer to FIG. 2, when the movable closed connector 22 is docked with the first needleless connector 23, the second engaging part 2313 is located in the second through hole 22115, and the hook part 22143 is fixed on a bottom end of the second engaging part 2313. The conduit 2212 compresses the second sealing part 2332, the second sealing part 2332 compresses the second circumferential part 2331, so that the second sealing part 2332 is away from the second opening 23111, and the conduit 2212 passes through the slit 23321 and enters the second sealing part 2332. The first end of the upper shell 2311 is against an inner wall of the middle cylinder part 22112. The positioning part 2213 moves along the conduit 2212 in a direction away from the first needleless connector 23 and compresses the first circumferential part 2231, so that the second end of the first sealing part 2232 is separated from the first opening 22122, and the first channel 22121 communicates with the second channel 23311. An inner side of the lower cylinder 22113 abuts an outer side of the stabilizing structure 2314, so the lower shell 2312 will not rock in the lower barrel 22113.

As shown in FIG. 2, and refer to FIG. 5A, when the movable closed connector 22 is separated from the first needleless connector 23, the arm part 22141 is pressed so that the hook part 22143 is separated from the bottom end of the second engaging part 2313. The conduit 2212 is separated from the second sealing part 2332, so that the slit 23321 is tightly closed. The second sealing part 2332 is reset by the elastic force of the second circumferential part 2331 and closes the second opening 23111. The first end of the upper shell 2311 is separated from the middle cylinder part 22112. The positioning part 2213 and the first sealing part 2232 are reset by the elastic force of the first circumferential part 2231, so that the second end of the first sealing part 2232 closes the first opening 22122.

Preferably, the force with which the hook 22143 is fixed to the second engaging part 2313 is 96.14 Newton-meters. The condition for achieving the above force is: as shown in FIG. 5B, the hook 22143 protrudes from the height H1 of the arm 22141 must be between 1.53-1.62mm. Therefore, if the user wants to remove the movable closed connector 22 by pulling out, the pulling force must be greater than 96.14 Newton-meters, and the hook 22143 can be separated from the second engaging part 2313; if the user wants to use a less labor-saving method to remove the movable closed connector 22, the user can optionally press the arm 22141 so that the hook 22143 can disengage from the second engaging part 2313. The above technical features make it difficult to remove the hook 22143 from the second engaging part 2313. The arm 22141 must be pressed to make the hook 22143 disengage from the second engaging part 2313. Therefore, the following effects can be achieved: first, it can effectively prevent leakage which may occur due to restlessness or turning over and other related medical actions during use; second, it can effectively prevent separation due to excessive movements during use, and will not cause liquid leakage or blood backflow; third, after the movable closed connector 22 is separated from the first needleless connector 23, it can be disinfected with alcohol before assembly, so as to reduce the time and costs of medical staff; fourth, it is suitable for use when infusion is performed on patients who are normally able to be independent; fifth, it is suitable for use when the mental state is unstable or the elderly are receiving medical assistance. It is worth noting that without the above technical features, the movable closed connector 22 will easily fall off, which will extend the cost of additional medical equipment and medical staff, and even increase the risk of bacterial infection for patients under treatment; in addition, the medical effect cannot be expected, which may cause doubts about curing the patient.

In some embodiments, the force with which the hook 22143 is fixed to the second engaging part is less than 96.14 Newton-meters. The condition for achieving the above force is that the height H1 of the hook 22143 protruding from the arm 22141 is less than 1.53 mm. Therefore, if the user wants to remove the movable closed connector 22 by pulling out, the hook 22143 can be separated from the second engaging part 2313 with the pulling force less than 96.14 Newton-meters; if the user wants to use a less labor-saving method to remove the movable closing connector 22, the user can optionally press the arm part 22141 so that the hook 22143 can disengage from the second engaging part 2313. The above technical features give the first closed needleless connector module 21 the effect of being clampable and easy to fall off.

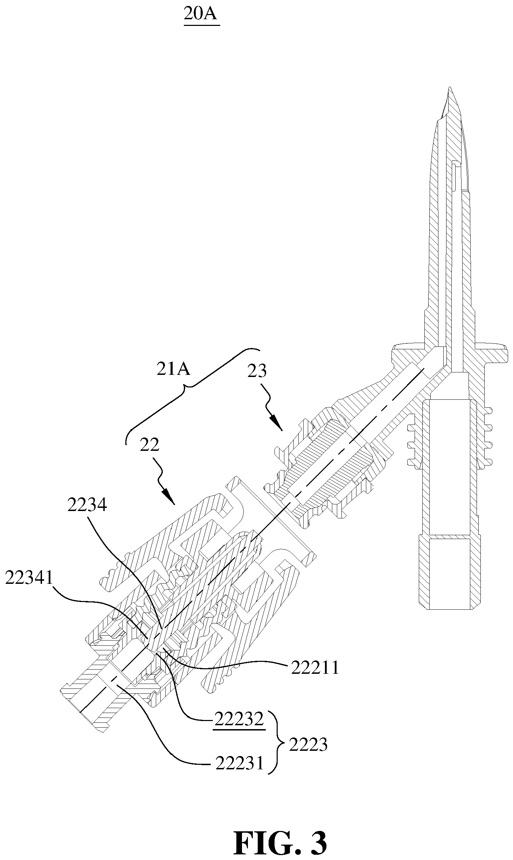

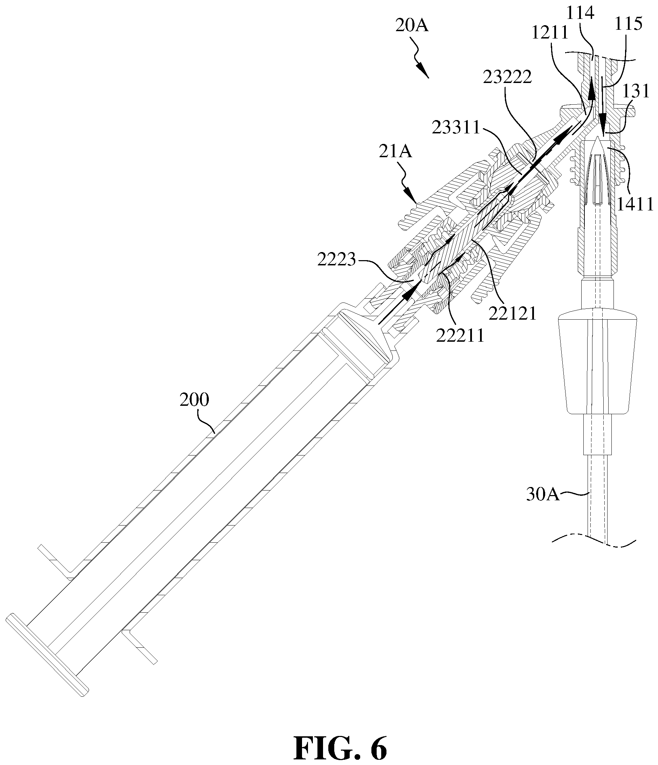

As shown in FIGS. 3 and 6, the first closed needleless connector module 21A differs from the first closed needleless connector module 21 in that: first, the second end 2222 is a Luer connector and is used to be combined with a syringe 200 (see FIGS. 1 and 6); second, the connecting channel 2223 includes a large-diameter part 22231 and a small-diameter part 22232; third, the first elastic valve 223 further includes a insertion rod 2234, a first end of the insertion rod 2234 is disposed at the first end of the first sealing part. A second end of the insertion rod 2234 has a head 22341. A diameter of the head 22341 is equal to a diameter of the small-diameter part 22232 and smaller than a diameter of the large-diameter part 22231. As shown in FIG. 6, when the movable closed connector 22 is docked with the first needleless connector 23, the head 22341 is located in the large-diameter part 22231 and forms a gap with a hole wall of the large-diameter part 22231, so that the connecting channel 2223 communicates with groove 22211. As shown in FIG. 3, when the movable closed connector 22 is separated from the first needleless connector 23, the head 22341 is located in the small-diameter part 22232 and maintains a gap with an inner side of the small-diameter part 22232 to keep the connecting channel 2223 flowing. Except for the above, the rest of the technical features of the second medicine mixing device 20A are exactly the same as those of the second medicine mixing device 20.

As shown in FIGS. 1 and 5A, the puncturing part 11 of the second medicine mixing device 20A passes through the blocking part 1412 of the second medicine mixing device 20, so that the second medicine mixing device 20 and the second medicine mixing device 20A can be connected in series.

In some embodiments, a plurality of second medicine mixing devices 20, 20A can be connected in series with each other in the above-mentioned manner, or a plurality of second medicine mixing devices 20 can be connected in series in the above-mentioned manner, or a plurality of second medicine mixing devices 20A can be connected in series in the above-mentioned manner, which are all possible implementations. In some embodiments, the infusion system may also only include the second medicine mixing device 20 or the second medicine mixing device 20A.

As shown in FIG. 1, a first end of the infusion drip set 30 passes through the blocking part 1412 of the first mixing device 10, thereby allowing the infusion drip set 30 to communicate with the second flow channel 131 through the third flow channel 1411 of the first mixing device 10. A first end of the infusion drip set 30A passes through the blocking part 1412 of the second medicine mixing device 20A, thereby allowing the infusion drop set 30A to communicate with the second flow channel 131 through the third flow channel 1411 and the second medicine mixing device 20A.

As shown in FIG. 1, the dispenser 40 includes a gathering part 41, three input terminals 42, 43, 44, an output terminal 45, a second closed needleless connector module 21B, and a screw-coupling needleless connector module 46. The input terminals 42, 43 and 44 are connected to a first end of the gathering part 41. The output terminal 45 is connected to a second end of the gathering part 41. The second closed needleless connector module 21B differs from the first closed needleless connector module 21A in that: first, the first elastic valve 223 does not include the insertion rod 2234; second, the second end 2222 is connected to a second end of the infusion drip set 30; and third, the second end 2322 is coupled to the input terminal 42. The screw-coupling needleless connector module 46 includes a screw-coupling part 461 and a second needleless connector 23A. The screw-coupling part 461 is disposed at a second end of the infusion drip set 30A. The second needleless connector 23A has the same structure as that of the first needleless connector 23. The upper shell 2311 of the second needleless connector 23A is screwed to the screw-coupling part, and the second end 2322 of the second needleless connector 23A is disposed at the input terminal 43. The input terminal 44 is disposed at a second end of the infusion drip set 30B. The operation of the second closed needleless connector module 21B is the same as that of the first closed needleless connector module 21A.

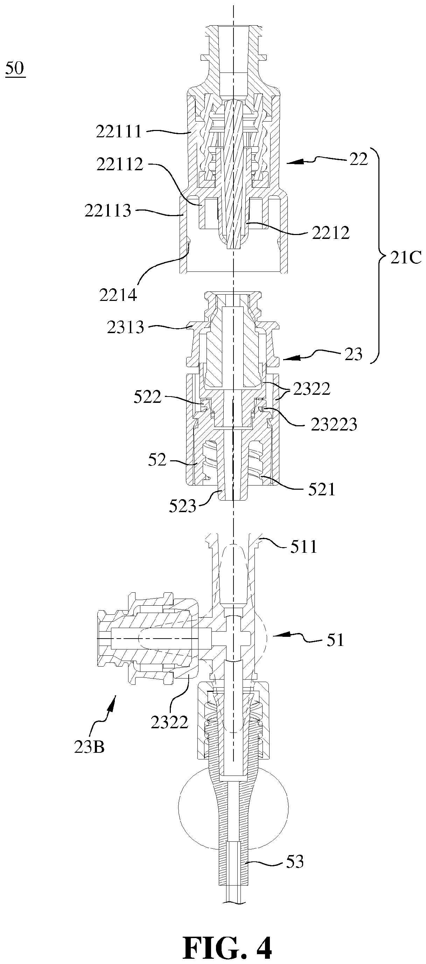

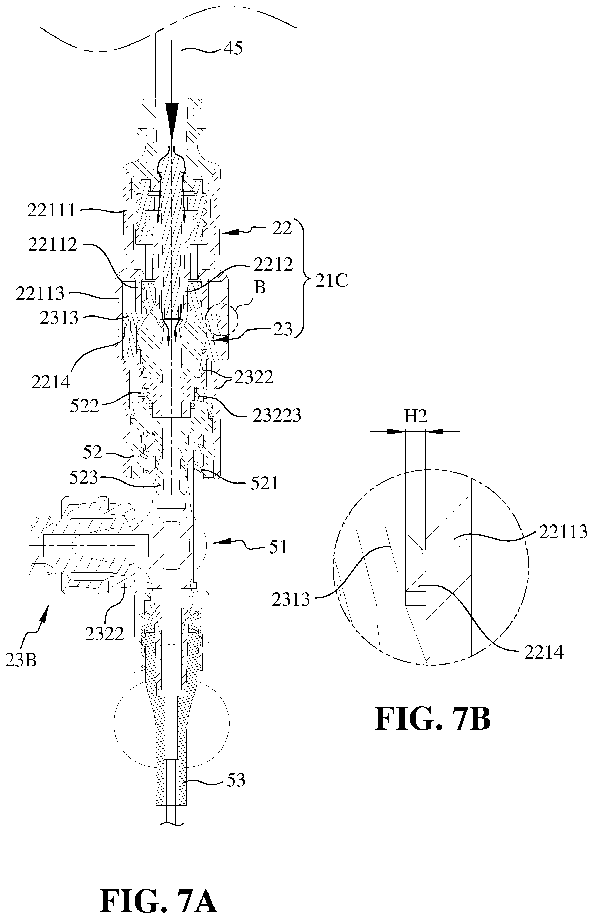

As shown in FIGS. 4, 7A and 7B, the separator 50 includes a valve body 51, a screw-coupling structure 52, an output terminal 53, a third needleless connector 23B and a third closed needleless connector module 21C. The valve body 51 is a three-way valve. The internal thread 521 of the screw-coupling structure 52 is threaded to the external thread 511 of a first end of the valve body 51. The screw-coupling structure 52 has an inner conduit 523. A first end of the output terminal 53 is disposed at a second end of the valve body 51, the second end part 2322 is disposed at a third end of the valve body 51. The third closed needleless connector module 21C differs from the first closed needleless connector module 21A in that: first, the middle cylinder part 22112 is disposed at an inner side of the first end of the upper tube part 22111, and the lower tube part 22113 is disposed on an outer side of the first end of the upper tube part 22111, the middle cylinder part 22112 extends inside the lower tube part 22113; second, the conduit 2212 extends from the inside of the upper tube part 22111 through the inside of the middle cylinder part 22112 to the lower tube part 22113; third, the first engaging part 2214 is a bump, which is disposed on an inner side of the lower tube part 22113 and is located below the middle cylinder part 22112; fourth, the second end part 2232 is disposed on the screw-coupling structure 52, the outer surface of a first end of the screw-coupling structure 52 has a plurality of pawls 522, a second end of the screw-coupling structure 52 has an internal thread 521, the bottom end of the second end part 2322 is a ratchet with an inner side surface having a plurality of tooth parts 23223; the structure of the third needleless connector 23B is exactly the same as that of the first needleless connector 23; fifth, the third opening 23222 communicates with the inner conduit 523. As shown in FIG. 7, when the movable closed connector 22 is docked with the first needleless connector 23, the first engaging part 2214 is fixed on a bottom end of the second engaging part 2313, and the force range of fixing the first engaging part 2214 to the second engaging part 2313 is between 1.4-8.1 Newton-meters (i.e., 1 and 6 pounds per foot), and the first channel 22121, the second channel 23311, the inner conduit 523 communicate with one another. As shown in FIG. 4, when the movable closed connector 22 is separated from the first needleless connector 23, the first engaging part 2214 is separated from the bottom end of the second engaging part 2313. Except the above, the third closed needleless connector module 21C has the same technical features as the first closed needleless connector module 21A.

As shown in FIG. 1, the retention needle 60 is disposed at a second end of the output terminal 53.

The following describes how the infusion system performs a medicine mixing procedure to evenly mix the high-concentration medicine liquid in the two vial bottles 100 and 100A and a syringe 200 and the diluting solution in the two infusion liquid bags 300 and 300A.

Step (1), according to the doctor's prescription, take out the vial bottles 100 and 100A containing a certain powder ingredient; according to the doctor's prescription, take out the infusion liquid bags 300 and 300A containing the diluting solution, the diluting solution can be saline or glucose solution; as shown in FIG. 1, the vial bottle 100 is sleeved on the integral vial adaptor 122 of the first medicine mixing device 10, and the puncturing member 1221 of the first medicine mixing device 10 puncture the cap 110 of the vial bottle 100; as shown in FIG. 5A, the vial bottle 100A is sleeved on the second end 2222 of the closed needleless connector module 21, and the puncturing member 1221 of the closed needleless connector module 21 puncture the cap 110 of the vial bottle 100A; and as shown in FIG. 1 and FIG. 5A, a first end of the puncturing part 11 punctures a film of an infusion tube of the infusion liquid bags 300, 300A, and the infusion drip set 30B directly punctures a film of an infusion tube of the infusion liquid bag 300B.

Step (1-1), the infusion liquid bags 300, 300A are at the top, and the infusion system is at the bottom; the infusion liquid bag 300 is squeezed, and the diluting solution in the infusion liquid bag 300 passes through the U-shaped through hole 111 of the first mixing device 10, the first long hole 114, the first flow channel 1211, and the puncturing member 1221 to enter the inside of the vial bottle 100; the infusion liquid bag 300A is at the top, and the infusion system is at the bottom. Squeeze the infusion liquid bag 300A, and the diluting solution in the infusion liquid bag 300A passes through the U-shaped through hole 111, the first long hole 114, the first flow channel 1211, the third opening 23222, the second channel 23311, the first channel 22121, the through holes 2233, the groove 22211, and the connecting channel 2223 and the puncturing member 1221 of the second chemical mixing device 20 to enter the inside of the vial bottle 100A; and shakes the vial bottles 100 and 100A to slightly mix the powder and diluting solution inside the vial bottles 100 and 100A to obtain a high-concentration medicinal liquid.

Step (2), the infusion liquid bags 300, 300A are at the bottom, and the infusion system is at the top; the infusion liquid bag 300 is squeezed, and the air in the infusion liquid bag 300 passes through the U-shaped through holes 111, the first long hole 114, the first flow channel 1211 and the puncturing member 1221 of the first mixing device 10 in sequence to enter the inside of the vial bottle 100; and the infusion liquid bag 300A is squeezed, the air in the infusion liquid bag 300A passes through the U-shaped through hole 111, the first long hole 114, the first flow channel 1211, the third opening 23222, the second channel 23311, the first channel 22121, the through holes 2233, the groove 22211, the connecting channel 2223 and the puncturing member 1221 of the second medicine mixing device 20 to enter the inside of the vial bottle 100A .

Step (3), loosen the infusion liquid bag 300, and the high-concentration medicinal liquid in the vial bottle 100 passes through the puncturing member 1221, the first flow channel 1211, the long hole 114 and the U-shaped through hole 111 of the first medicine mixing device 10 in sequence to enter the inside of the infusion liquid bag 300; as shown in FIG. 5A, loosen the infusion liquid bag 300A, and the high-concentration medicinal liquid in the vial bottle 100A passes through the puncturing member 1221, the connecting channel 2223, the groove 22211, the through holes 2233, the first channel 22121, the second channel 23311, the third opening 23222, the first flow channel 1211, the first long hole 114 and the U-shaped through hole 111 of the second medicine mixing device 20 in sequence to enter the inside of the infusion liquid bag 300A; as shown in FIG. 6, the high-concentration medical liquid in the syringe 200 passes through the connecting channel 2223, the groove 22211, the through holes 2233, the first channel 22121, the second channel 23311, the third opening 23222, the first flow channel 1211, the first elongated hole 114 and the U-shaped through hole 111, the third flow channel 1411, the second flow channel 131, the two long holes 115 and the side holes 112 of the second medicine mixing device 20A in sequence to enter the infusion liquid bag 300A.

By repeatedly performing steps (2) and (3), all the high-concentration medicinal liquids in the vial bottles 100, 100A and syringes 200 can enter the inside of the infusion liquid bags 300, 300A, and at the same time, the high-concentration medicine solution in the infusion liquid bags 300 and 300A and the diluting solution are thoroughly mixed to obtain the diluted medicine.

It is worth noting that during the execution of the medicine mixing procedure, because the distance D1 from the U-shaped through hole 111 to the sharp end of the puncturing part 11 is smaller than the distance D2 from the side hole 112 to the sharp end of the puncturing part 11, and at the same time, the larger and longer the diameter of the puncturing part 11 is, the gap in the infusion tube of the infusion liquid bag 300, 300A of the puncturing of the puncturing part 11 is smaller, the flow space is smaller, and a tension is generated to block the medical solution, thereby preventing high-concentration medicinal liquid from entering the side holes 112.

The following will describe how the infusion system performs the infusion procedure, thereby infusing the diluted medicinal liquid into the patient's body.

Step (a), as shown in FIG. 1, the infusion liquid bags 300, 300A, 300B are at the top, and the infusion system is at the bottom; the diluted medicine in the infusion liquid bag 300 passes through the side holes 112, the second long holes 115, the second flow channel 131, the third flow channel 1411, the infusion drip set 30 of the medicine mixing device 10 and the connecting channels 2223, the groove 22211, the through holes 2233, the first channel 22121, the second channel 23311, the third opening 23222 and the input terminal 42 of the closed needleless connector module 21A to enter the inside of the gathering part 41; as shown in FIGS. 1, 5A and 6, the diluted medicine in the infusion liquid bag 300A passes through the side holes 112, the second long holes 115, the second flow channel 131, the third flow channel 1411 of the second medicine mixing device 20, the side holes 112, the second elongated holes 115, the second flow channel 131, the third flow channel 1411, the infusion drip sleeve 30A of the second medicine mixing device 20A, the screw-coupling part 461 of the screw-coupling needless connector module 46, the slit 23321, the second channel 23311, the third opening 23222 and the input terminal 43 of the needleless connector to enter the inside of the gathering part 41; and as shown in FIG. 1, other medicines in the infusion liquid bag 300B pass through the infusion drip set 30B and the input terminal 44 in sequence to enter the inside of the gathering part 41.

Step (b), as shown in FIGS. 1 and 7A, after the three medicines are mixed inside the gathering part 41, the mixed medicine passes through the output terminal 45 and then enter the third closed needleless connector module 21C.

Step (c), as shown in FIGS. 1 and 7A, the mixed medicine passes through the connecting channel 2223, the groove 22211, the through holes 2233, and the first channel 22121, the second channel 23311, the third opening 23222 of the closed needleless connector module 21C, the screw-coupling structure 52, the valve body 51, the output end 53 of the separator 50, and the retention needle 60 in sequence to enter the patient's body.

Thus, because the U-shaped through hole 111 and the side holes 112 are separated by the first partition 113, and the first flow channel 1211 and the second flow channel 131 are separated by the second partition 15, in step (3), the high-concentration medicinal solution in the vial bottle 100 will only pass through the puncturing member 1221, the first flow channel 1211, the first long hole 114 of the puncturing part 11, and the U-shaped through hole 111 of the first medicine mixing device 10 in sequence, instead of passing through the second flow channel 131, the second long holes 115 and the side holes 112 of the first medicine mixing device 10 in sequence; and the high-concentration medicinal liquid in the vial bottle 100A will only pass through the puncturing member 1221, the connecting channel 2223, the groove 22211, the through holes 2233, the first channel 22121, the second channel 23311, the third opening 23222, the first flow channel 1211, the first long hole 114 and the U-shaped through hole 111 of the second chemical mixing device 20, instead of passing through the second flow channel 131, the second elongated holes 115 and the side holes 112 of the second chemical mixing device 20 in sequence. Therefore, when the high-concentration medicinal liquid in the vial bottles 100 and 100A passes through the U-shaped through hole 111 of the first medicine mixing device 10 and the second medicine mixing device 20, there will not be any high-concentration medicinal liquid attached to the inner side walls of the side holes 112. When step (2) is performed again, since there is no high-concentration medicinal liquid attached to the inner walls of the side holes 112, even if the air in the infusion liquid bags 300 and 300A passes through the side holes 112, there will not be any high-concentration liquid medicine remaining in the space between the second flow channel 131 and insertion hole 132.

Since there is no high-concentration liquid medicine remaining in the space between the second flow channel 131 and the insertion hole 132, during the infusion process, only the diluted medicine can pass through the side holes 112, the second long hole 115, the second flow channel 131, the third flow channel 1411, the infusion drip set 30, 30A, the dispenser 40, the separator 50, and the retention needle 60 in sequence to enter the patient's body, the problem of residual high-concentration medicinal liquid entering the patient's body will not occur; thereby, the patient will not feel uncomfortable, and it is very safe and reliable.

Furthermore, because there is no high-concentration medical liquid remaining in the space between the second flow channel 131 and the insertion hole 132, the nursing staff does not need to discharge any residual high-concentration medicinal liquid, so that the infusion can achieve the prescribed dosage of medicine in the patient's body without any reduction and is consistent with the doctor's prescription, thereby maintaining the therapeutic effect and causing no medicine waste.

In addition, the first long hole 114 only allows the high-concentration medicinal liquid in the vial bottles 100 and 100A to pass into the infusion liquid bags 300 and 300A. The second long holes 115 only allow the diluted medicine in the infusion liquid bags 300 and 300A into the patient's body so as to prevent the diluted medicine from flowing back into the vial bottles 100 and 100A. As such, the dose infused into the patient's body is not reduced and is consistent with the doctor's prescription, thereby maintaining the therapeutic effect without causing any medicine waste.

Moreover, regarding the first closed needleless connector modules 21, 21A, when the movable closed connector 22 and the first needleless connector 23 are separated, both the movable closed connector 22 and the first needleless connector 23 have a sealing effect and can prevent liquid leakage. Therefore, the vial bottle 100A and the syringe 200 can be removed from the movable closed connector 22 so that the second medicine mixing device 20, 20A can be reused. If medical staff want to mix medicine a plurality of times, the same second medicine mixing device 20, 20A can be reused to install a plurality of vial bottles 100A or a plurality of syringes 200 in batches to mix medicine a plurality of times without replacing the second medicine mixing device 20 and 20A, and thereby the cost is saved. In practice, medical staff can also connect a plurality of second medicine mixing devices 20 and 20Ain series, install a plurality of vial bottles 100A or a plurality of syringes 200 at one time, and complete the above-mentioned plurality of medicine mixing procedures at once, and thereby saving time. It should be noted that each time before installing the vial bottle 100A or the syringe 200, the movable closed connector 22 and the first needleless connector 23 must be sterilized.

In addition, regarding the second closed needleless connector module 21B, when the movable closed connector 22 is separated from the first needleless connector 23, both the movable closed connector 22 and the first needleless connector 23 have the sealing effect to prevent liquid leakage.

Moreover, regarding the third closed needleless connector module 21C, the force range of fixing the first engaging part 2214 to the second engaging part 2313 is between 104-801 Newton-meters (i.e., 1 and 6 pounds per foot), which allows easy separation. Even if the patient moves erratically, the movable closed connector 22 and the first needleless connector 23 can be immediately separated to prevent leakage of medical solution and blood. In addition, alcohol pads can be used for disinfection and reuse without replacement. When the heights H2, H3, and H4 of the first engaging part 2214 protruding from the lower tube part 22113 are 0.05 mm, the force of fixing the first engaging part 2214 to the second engaging part 2313 is 2.7 Newton-meters (i.e., 2 pounds per foot). When the heights H2, H3, and H4 of the first engaging part 2214 protruding from the lower tube part 22113 are 0.07 mm, the force of fixing the first engaging part 2214 to the second engaging part 2313 is 4.1 Newton-meters. When the heights H2, H3, and H4 of the first engaging part 2214 protruding from the lower tube part 22113 are 0.1 mm, the force of fixing the first engaging part 2214 to the second engaging part 2313 is 5.4 Newton meters (i.e., 4 pounds per foot). When the heights H2, H3, and H4 of the first engaging part 2214 protruding from the lower tube part 22113 are 0.14 mm, the force of fixing the first engaging part 2214 to the second engaging part 2313 is 8.1 Newton-meters (i.e., 6 pounds per foot).

It is worth noting that since the groove wall of the groove 22211 is arc-shaped, the medical liquid can flow along the arc-shaped groove wall of the groove 2221 to prevent the generation of eddy currents and thereby increase the flow rate of the medical liquid.

It is also worth noting that because the diameter of the groove 22211 is larger than the diameter of the first sealing part 2232, even if the first circumferential part 2231 is compressed and deformed and the first sealing part 2232 is close to the groove 22211, the groove 22211 remains open to these through holes 2233 and will not be affected.

In addition, the upper shell 2311 of the third needleless connector 23B is a Luer connector and is used to combine with a syringe 200. Thereby, the medical staff can take out the blood sample from or add non-toxic general medicine to the second end of the valve body 51 through the third needleless connector 23B of the separator 50.

In some embodiments, the valve body 51 may also be a female Luer connector.

When the first needleless connector 23 rotates along a first direction by a first rotation force, the first needleless connector 23 will transmit the first rotation force sequentially through the teeth 23223 and the pawls 522 to the screw-coupling structure 52. The screw-coupling structure 52 is pushed by the first rotation force and rotates along the first direction, so that the screw-coupling structure 52 is fixed on the first end of the valve body 51. After the screw-coupling structure 52 is fixed on the first end of the valve body 51, when the first needleless connector 23 rotates along a second direction opposite to the first direction by a second rotation force, the first needleless connector 23 uses the second rotation force to squeeze the pawls 522 through the teeth part 23223, causing the pawls 522 to bend and deform to break away from the teeth part 23223, and the first needleless connector 23 rotates idlingly with respect to the screw-coupling structure 52 along the second direction. At this time, the screw-coupling structure 52 is still fixed to the first end of the valve body 51, and the first needleless connector 23 is completely unable to drive the screw-coupling structure 52 to rotate in the second direction.

After the screw-coupling structure 52 is fixed on the first end of the valve body 51, when the first needleless connector 23 rotates in direction around the first end of the valve body 51 by a third rotation force, the first needleless connector 23 transmits the third rotation force to the pawls 522 through the tooth parts 23223. The third rotation force is greater than the force that the pawls 522 can withstand, and the pawls 522 are broken, so that the screw-coupling structure 52 can rotate arbitrarily along the first direction and the second direction with respect to the first needleless connector 23, so that the liquid medicine in the output terminal 53 can maintain smooth flow.

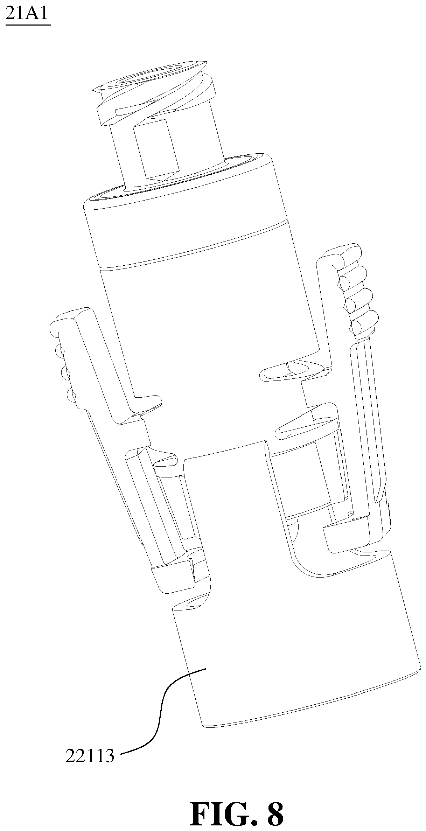

As shown in FIG. 8, the difference between the first closed needleless connector module 21A1 and the first closed needleless connector module 21A is: the length of the lower tube part 22113 is longer.

As shown in FIG. 9A and FIG. 9B, the structural difference between the third closed needleless connector module 21D and the third closed needleless connector module 21C is: first, the sleeve 2211A of the movable closed connector 22A only includes an upper tube part 22111A and a lower tube part 22113A; second, the second coupling part 232A is integrally formed with the screw-coupling structure 52; third, the first needleless connector 23C includes a communication tube 234, and the communication tube 234 is provided in the shell and includes an upper cylinder 2341, a large-diameter part 2342 and a lower cylinder 2343. The upper cylinder 2341 is arranged below the large-diameter part 2342, and its top is a closed end, and a through hole is provided (not shown in the drawings), the through hole penetrates one side wall of the upper cylinder 2341 and communicates with the inside of the upper cylinder 2341. The lower cylinder 2343 is arranged below the large-diameter part 2342, and its bottom end is an open end. The inside of the upper cylinder 2341, the inside of the large-diameter part 2342 and the inside of the lower cylinder 2343 communicate with one another; fourth, the first needleless connector 23C includes a third elastic valve 235. The third elastic valve 235 is provided in the shell 231A and has a first circumferential parts 2351 and a third sealing part 2352. The third circumferential part 2351 is arranged around the outside of the upper cylinder 2341 with bottom end abutting against the large-diameter part 2342. The third sealing part 2352 is provided on the top of the third circumferential part 2351 and a slit 2353 is provided; fifth, the first needleless connector 23C includes a fourth elastic valve 236. The fourth elastic valve 236 is provided in the shell 231A and has a fourth circumferential part 2361 and a fourth sealing part 2362. The fourth circumferential part 2361 is arranged around the outside of the lower cylinder 2343 and its top end is against the large-diameter part 2342. The fourth sealing part 2362 is arranged at the bottom end of the fourth circumferential part 2361 and is against an inner wall of the second coupling part 232A, the lower shell 2312A presses the top of the fourth sealing part 2362, the fourth sealing part 2362 is provided with a slit 2363, and the slit 2363 of the fourth sealing part 2362 is aligned downward with the inside of the lower cylinder 2343 and the third opening 23222; sixth, two grooves 22116 are opened in the lower tube part 22113A.

As shown in FIG. 9C, FIG. 9D and FIG. 9E, the difference in the docking methods of the third closed needleless connector module 21D and the third closed needleless connector module 21C is: first, the upper cylinder 2341 supports pushes to open the slit 2353 of the third sealing part 2352, the upper cylinder 2341 pushes against and compresses the first sealing part 2232, the upper cylinder 2341 passes through the first opening 22122 and enters the inside of the conduit 2212, and the conduit 2212 compresses the third sealing part 2352, the third sealing part 2352 compresses the third circumferential part 2351 so that the through hole of the upper cylinder 2341 is exposed outside the third sealing part 2352 and is located inside the conduit 2212; second, the third circumferential part 2351 pushes against the large-diameter part 2342, the large-diameter part 2342 compresses the fourth circumferential part 2361, and the lower cylinder 2343 pushes to open the slit 2363 of the fourth sealing part 2362 and enters the third opening 23222, so that the first channel 22121, the communication tube 234 and the inner conduit 523 are connected..

As shown in FIG. 9A and FIG. 9B, the difference in the separation methods of the third closed needleless connector module 21D and the third closed needleless connector module 21C is that: first, the fourth circumferential part 2361 is pushed by its elastic force against the large-diameter part 2342, causing the communication tube 234 and the third elastic valve 235 to move, and the lower cylinder 2343 returns to the inside of the fourth circumferential part 2361, causing the slit 2363 of the fourth sealing part 2362 to be tightly closed; second, the third circumferential part 2351 pushes against the conduit 2212 through its elastic force to drive the movable closed connector 22A to move, so that the upper cylinder 2341 is separated from the conduit 2212 and returns to the third circumferential part 2351; third, the conduit 2212 is separated from the third sealing part 2352 so that the slit 2353 of the third sealing part 2352 is closed, and the third sealing part 2352 is reset by the elastic force of the third circumferential part 2351 and seals the second opening 23111; fourth, the upper shell 2311A is separated from the lower tube part 22113A, the first sealing part 2232 is reset by its elastic force, so that the second end of the first sealing part 2232 closes the first opening 22122.

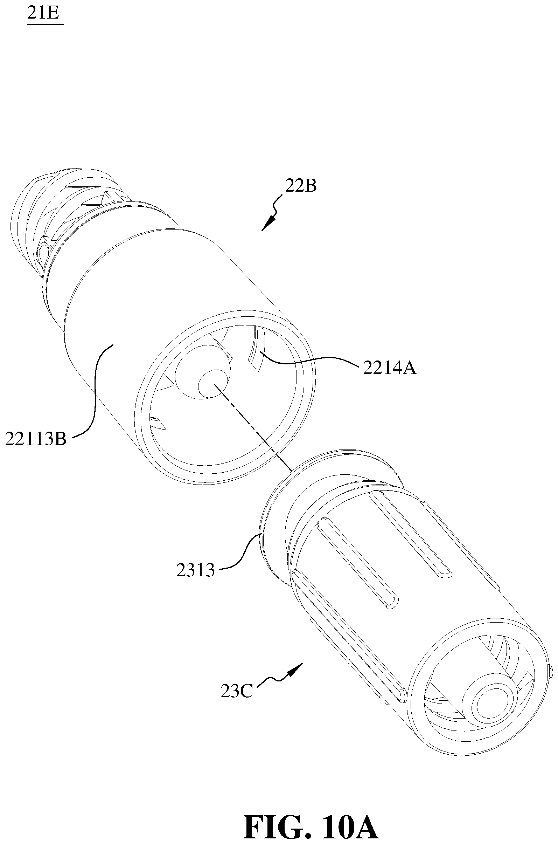

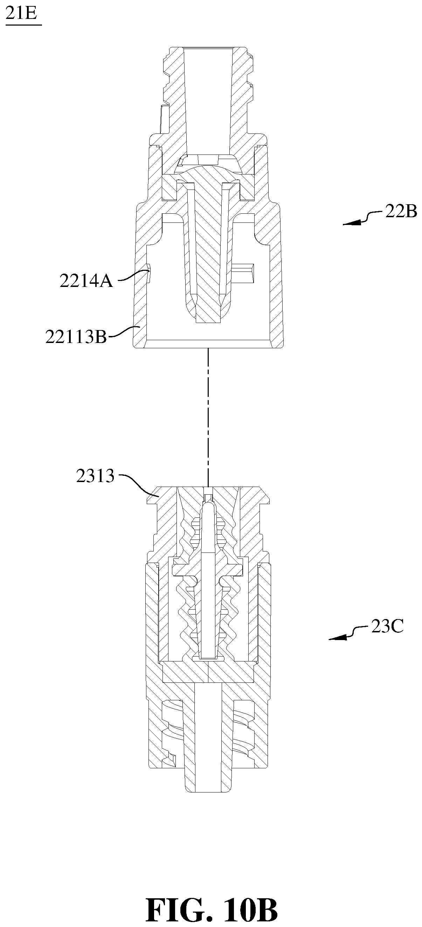

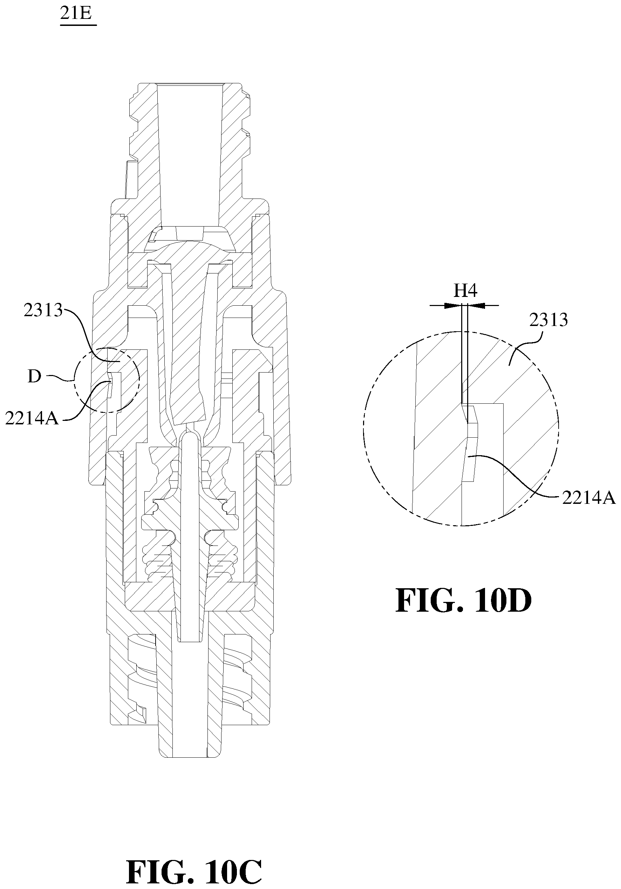

As shown in FIGS. 10A, 10B, 10C and 10D, the structural differences between the third closed needleless connector module 21E and the third closed needleless connector module 21D are: first, the first engaging part 2214A forms a plurality of bumps with shorter arc length arranged with intervals; second, the lower tube part 22113B of the movable closed connector 22B has no groove.

In some embodiments, there may be only one first engaging part 2214A.

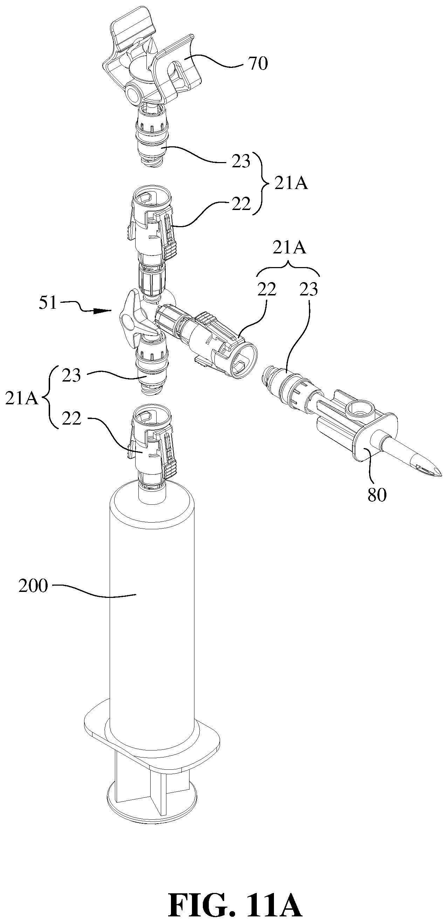

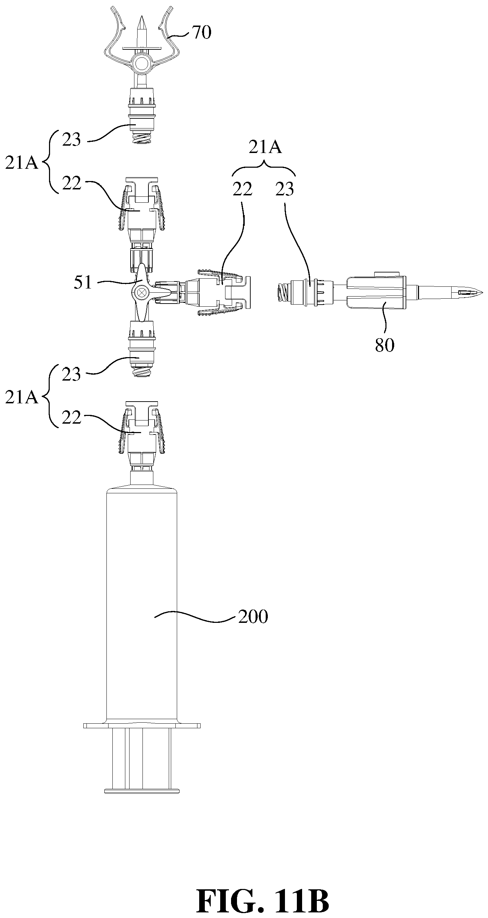

In some embodiments, as shown in FIGS. 11A and 11B, the two movable closed connectors 22 of the first closed needleless connector module 21A are respectively provided at one end of the syringe 200 and the first end and the second end of the valve body 51, the first needleless connector 23 of the first closed needleless connector module 21A is provided at the third end of the valve body 51, and a clamp 70 is provided at one end of one of the first needleless connectors 23, a puncture head 80 is provided at one end of the other first needleless connector 23. These embodiments are suitable for use in automated dispensing systems.

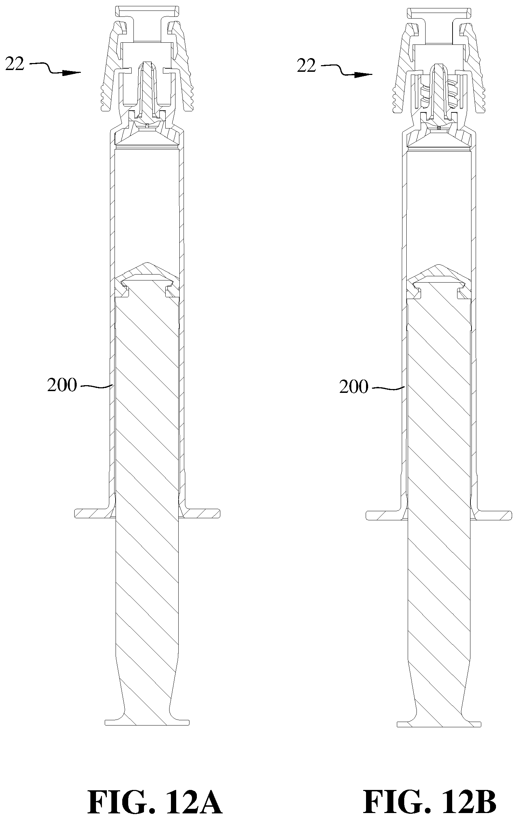

In some embodiments, as shown in FIGS. 12A and 12B, the syringe 200 is integrally formed with two movable closed connectors 22 respectively. Because the syringe 200 and the movable closed connectors 22 are integrally formed, a large number of combinations of the syringe 200 and the movable closed connectors 22 can be produced, reducing manufacturing costs, such as the waste of resources in the use of sterilization packaging bags and reducing the carbon emission caused by sterilization. The user can also assemble additional spare parts (for example, it can be applied to the combination of the syringe 200 and the movable closed connector 22 of various milliliter specifications) during use, which saves labor and time, and is unified, easy and convenient to use.

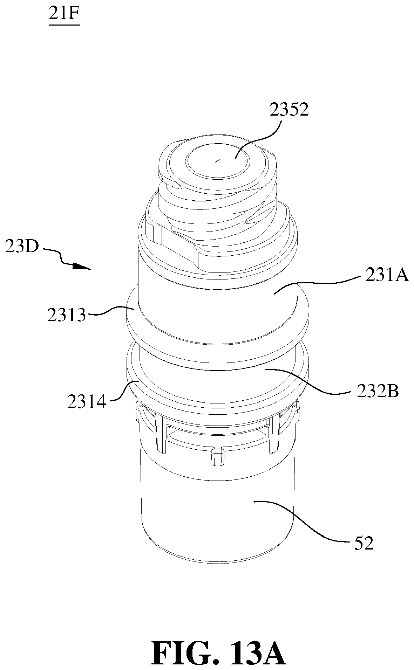

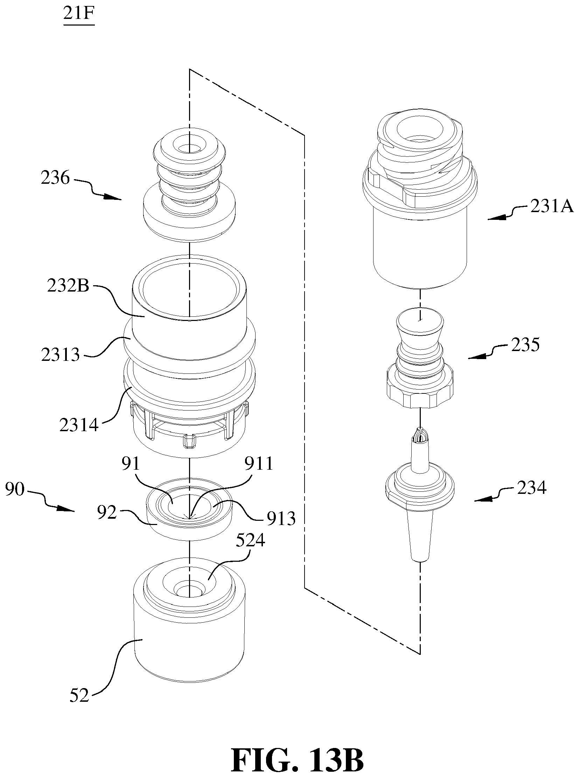

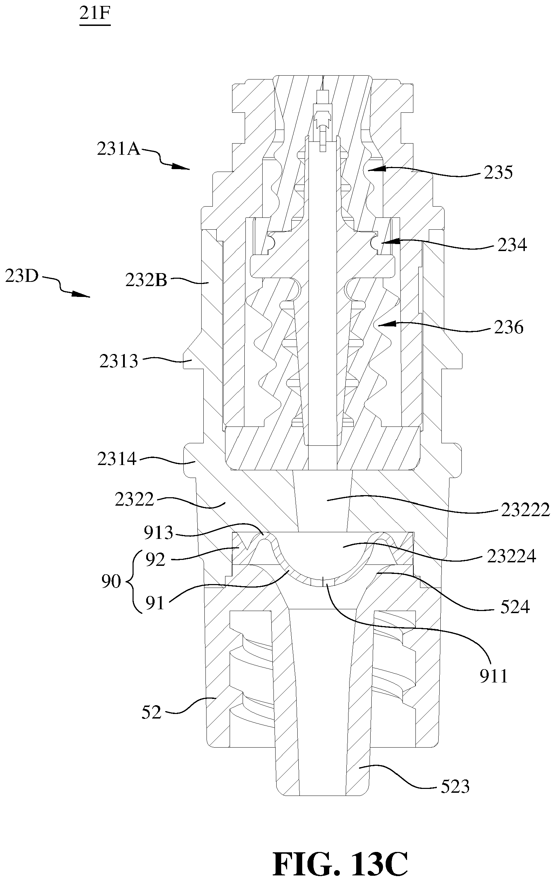

In some embodiments, as shown in FIGS. 13A, 13B and 13C, the structural differences between the third closed needleless connector module 21F and the third closed needleless connector module 21D are: first, there is a stabilizing structure 2314 on the outside of the second coupling part 232B; second, the second engaging part 2313 is provided on an outer side of the second coupling part 232B; third, the third closed needleless connector module 21F further includes a balance pressure valve 90 is provided between the second end 2322 of the second coupling part 232B of the first needleless connector 23D and the screw-coupling structure 52.

Preferably, the second end 2322 forms a chamber 23224, and a bowl-shaped groove 524 is formed in the top center of the screw-coupling structure 52. The balance pressure valve 90 is disposed in the chamber 23224 and includes a valve body 91 and a fixed part 92. The valve body 91 is in the shape of a semi-sphere and is located in the bowl-shaped groove 524. It has a slit 911 and a bending part 913 formed on the outside. The fixing part 92 is arranged around the outside of the bending part 913 of the valve body 91. The top of the fixing part 92 is against the bottom of the second end 2322. The side wall of the fixing part 92 is against the inner wall of the chamber 23224. The bottom of the fixing part 92 is against the top of the screw-coupling structure 52.

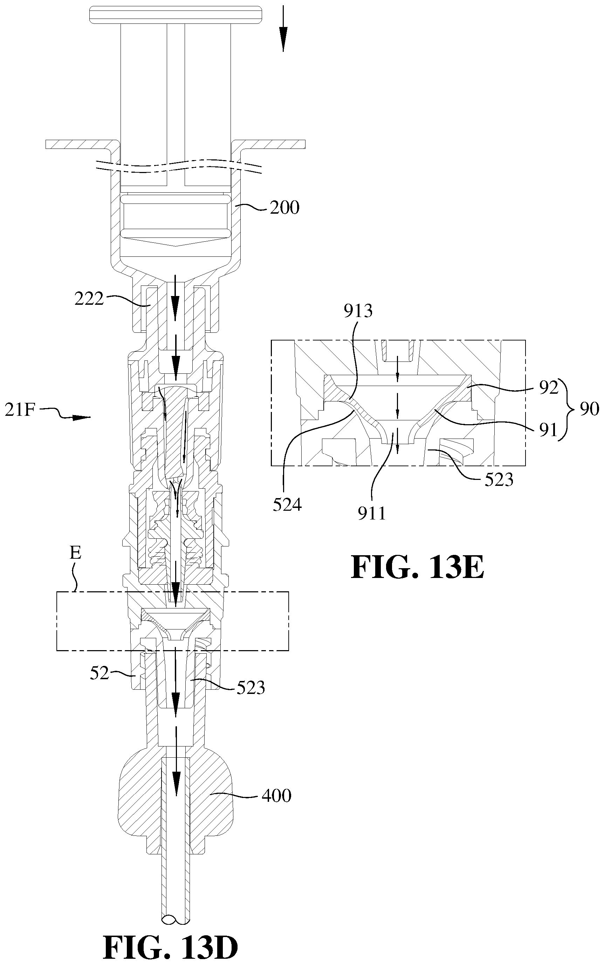

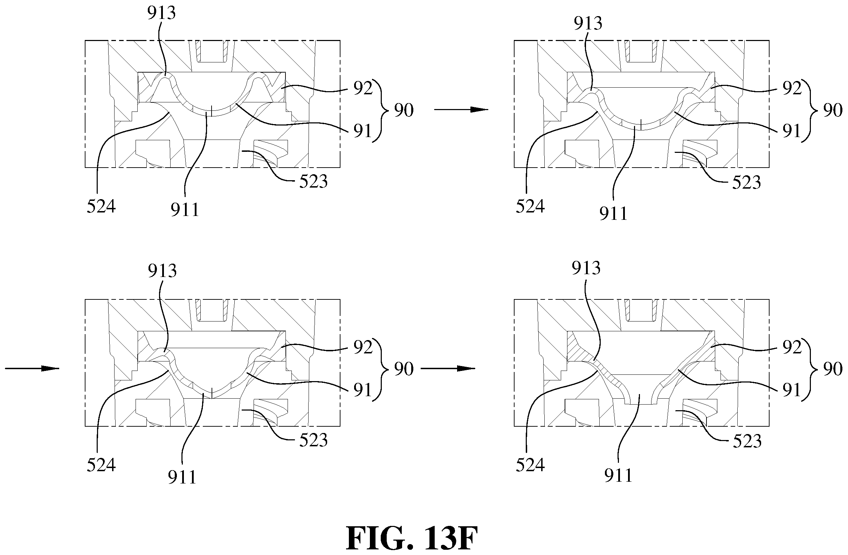

As shown in FIGS. 13D, 13E and 13F, the syringe 200 is connected to the first coupling part 222, a long tube 400 is connected to the screw-coupling structure 52, and the inner conduit 523 extends to the inside of the long tube 400. When the syringe 200 inputs the medical liquid into the human body through the separator 50 and the long tube 400, the syringe 200 provides positive pressure. Under the influence of the positive pressure, the medical liquid will be concentrated in the internal space of the semi-spherical valve body 91 and move to store more. If there is too much liquid, the bending part 913 of the semi-spherical valve body 91 cannot withstand the positive pressure and will be forced by the liquid and gradually move to open the slit 911, allowing the liquid to enter the long tube 400 through the slit 911 and the inner conduit 523.

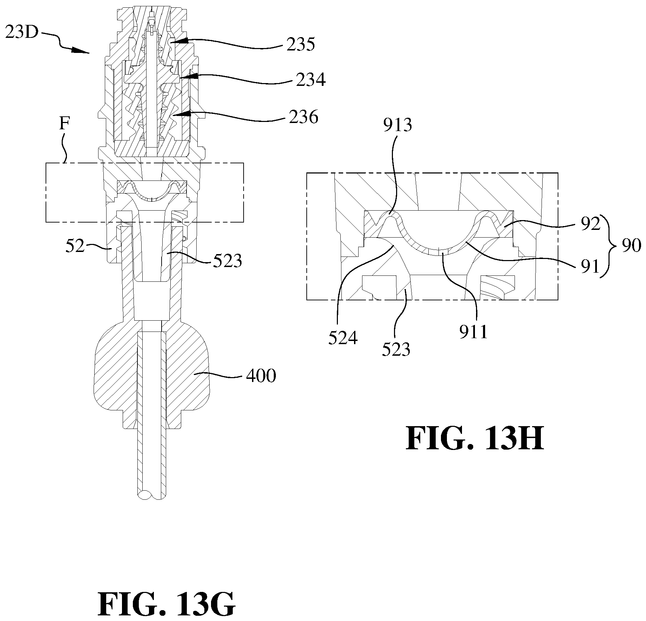

As shown in FIGS. 13G and 13H, after the syringe 200 is removed from the first coupling part 222, the communication tube 234, the third elastic valve 235 and the fourth elastic valve 236 are reset to provide negative pressure. As a result, during the resetting process of the third elastic valve 235 and the fourth elastic valve 236, the part of the semi-spherical valve body 91 that was originally filled with medical liquid still opens the slit 911 in the direction of the output end and flows out until it closes and reaches a stable pressure, and then seal the opening to prevent the medicine liquid and blood from backflowing.

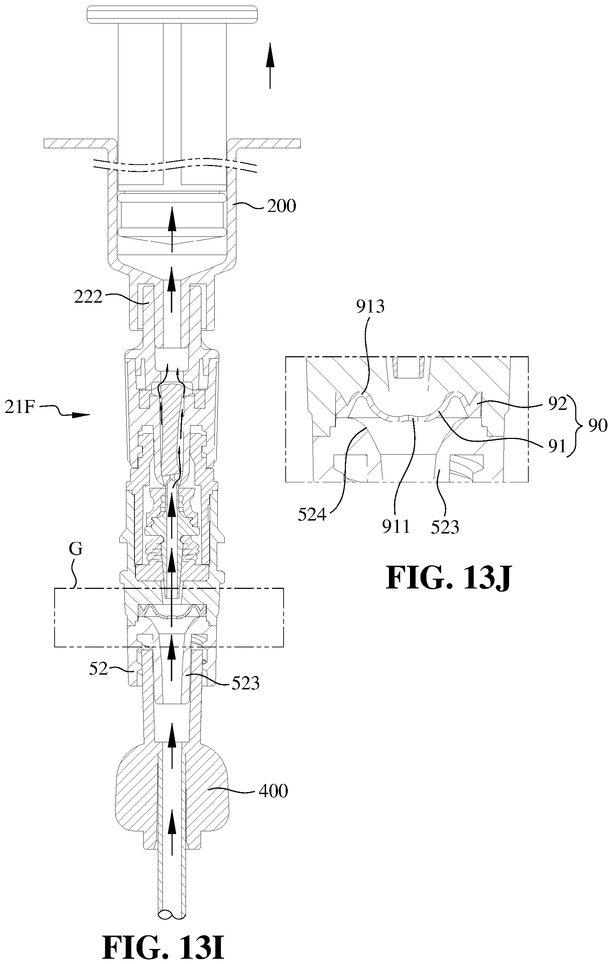

As shown in FIG. 13I and FIG. 13J, when the syringe 200 extracts excess medical liquid through the separator 50 and the long tube 400, the syringe 200 provides negative pressure, and the slit 911 will be affected by the negative pressure and open to supply the excess liquid. The medical solution or the blood sample will not be damaged during blood drawing and enters the syringe 200 through the separator 50.

In case the medicine bag falls during the infusion process, or when the patient coughs, or when the medicine bag is lower than the human body, the balance pressure valve 90 can prevent blood from backflowing.

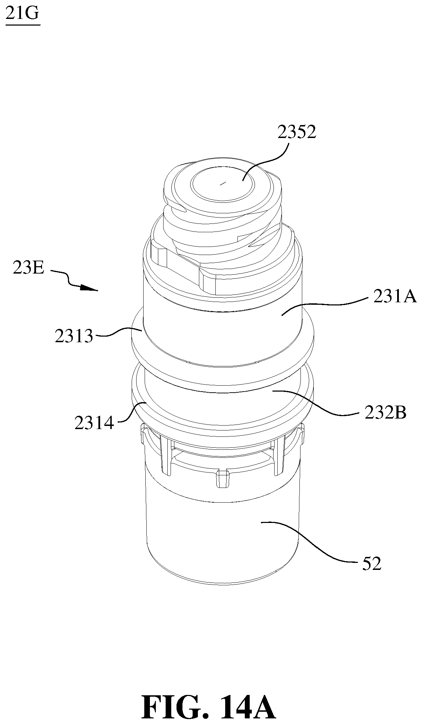

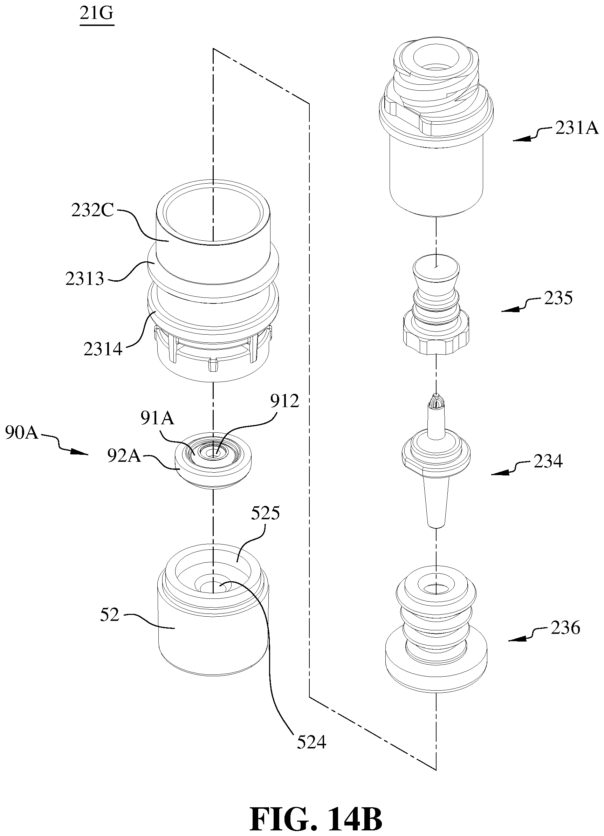

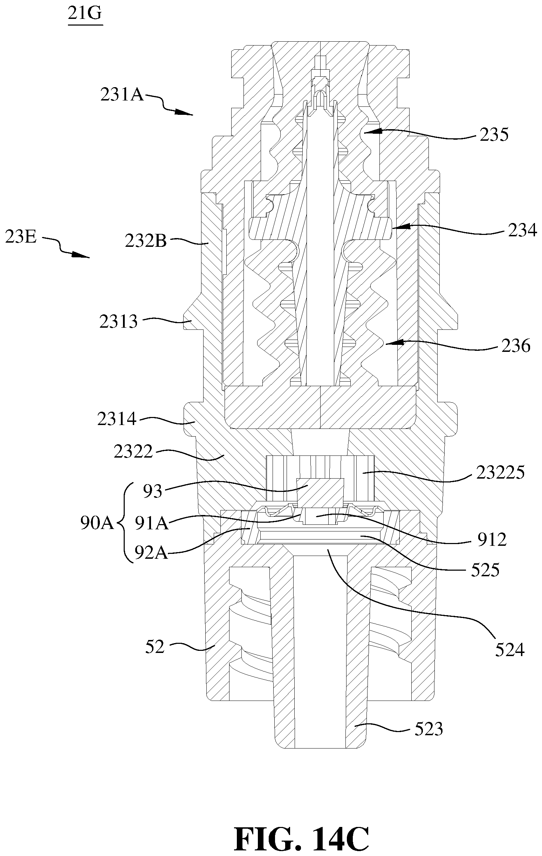

In some embodiments, as shown in FIGS. 14A, 14B and 14C, the structural difference between the third closed needleless connector module 21G and the third closed needleless connector module 21F is: a check valve 90A replaces the balanced pressure valve 90.

Specifically, the second end 2322 of the second coupling part 232C of the first needleless connector 23E forms an upper chamber 23225, the screw-coupling structure 52 forms a lower chamber 525, and the check valve 90A includes a valve body 91A, a fixing part 92A and a stopper 93. The valve body 91Ais located in the lower chamber 525 and has a through hole 912. The top of the fixing part 92 abuts against the bottom of the second end 2322 , the side wall of the fixing part 92 abuts against the inner wall of the lower chamber 525, and the bottom of the fixing part 92 abuts against the top of the screw-coupling structure 52. The stopper 93 is provided in the upper chamber 23225.

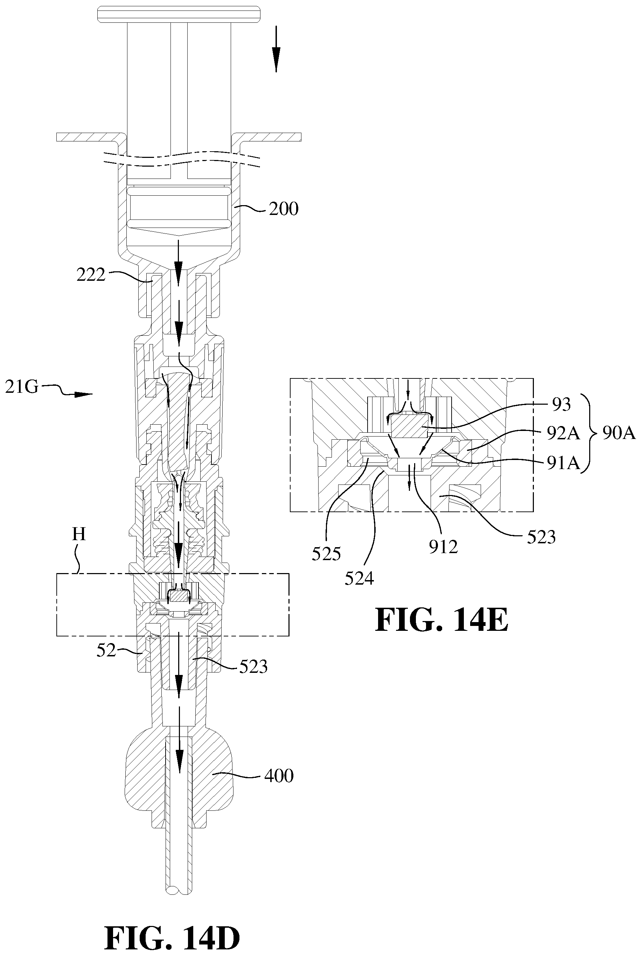

As shown in FIGS. 14D and 14E, the syringe 200 is connected to the first coupling part 222, the long tube 400 is connected to the screw-coupling structure 52, and the inner conduit 523 extends to the inside of the long tube 400. When the syringe 200 inputs the medical solution into the human body through the separator 50 and the long tube 400, the syringe 200 provides positive pressure, and the valve body 91A is affected by the positive pressure and moves away from the stopper 93, and the stopper 93 no longer closes the through hole 912. Therefore, the medical liquid enters the long tube 400 through the through hole 912 and the inner conduit 523.

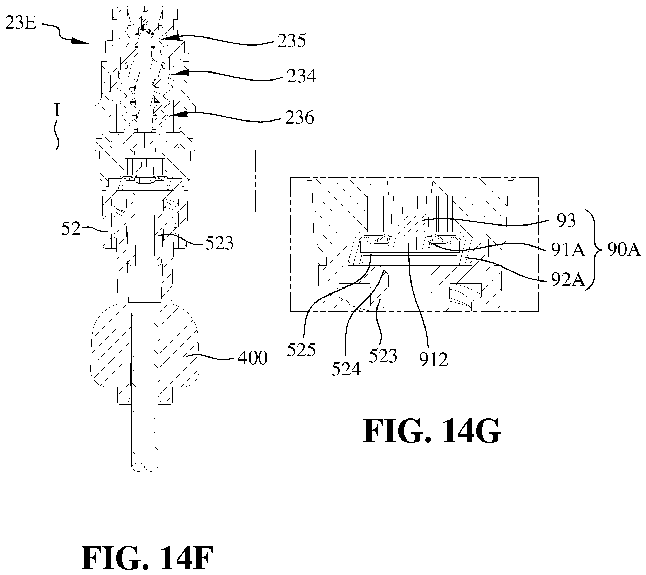

As shown in FIGS. 14F and 14G, after the syringe 200 is removed from the first coupling part 222, the communication tube 234, the third elastic valve 235 and the fourth elastic valve 236 are reset to provide negative pressure, and the valve body 91A is pressed against the stopper 93 due to the influence of the negative pressure, and the stopper 93 closes the through hole 912 to prevent the backflow of the medical solution and blood.

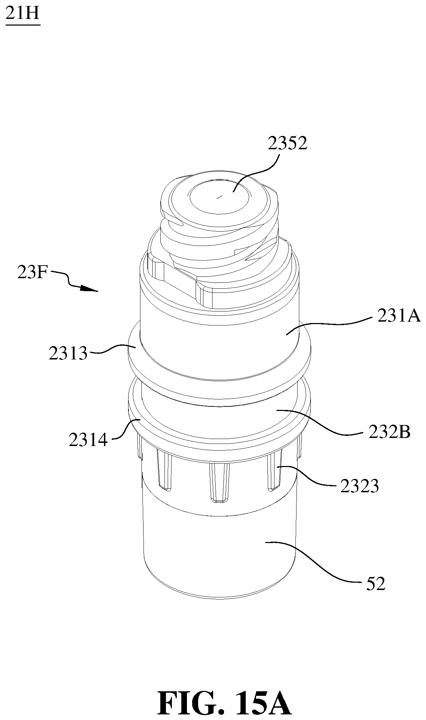

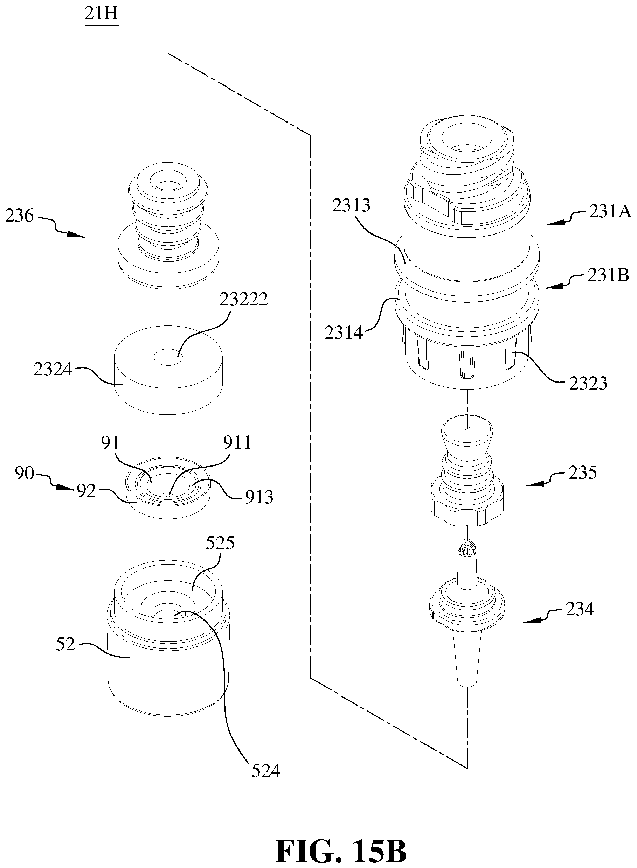

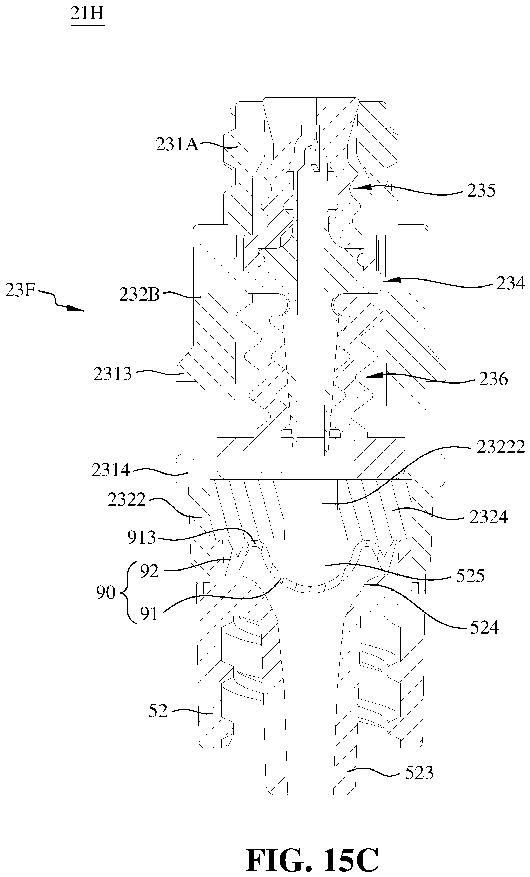

As shown in FIGS. 15A, 15B and 15C, the structural differences between the third closed needleless connector module 21H and the third closed needleless connector module 21F are: first, the shell 231A and the second coupling part 232B of the first needleless connector 23F are integrally formed; second, the outer wall of the second coupling part 232B has a plurality of anti-slip ribs 2323, which can prevent the user's hand from slipping; third, the second end 2322 of the second coupling part 232B has a block 2324 inside, and the block 2324 is disposed with the third opening 23222; fourth, the screw-coupling structure 52 forms a lower chamber 525, and the valve body 91 is located in the lower chamber 525, the top of the fixing part 92 abuts against the bottom of the block 2324, the side wall of the fixing part 92 abuts against the inner wall of the lower chamber 525, and the bottom of the fixing part 92 abuts against the top of the screw-coupling structure 52. In some embodiments, the balance pressure valve 90 of the third closed needleless connector module 21H may be replaced by a check valve 90A.

In some embodiments, the check valve 90A replaces the balanced pressure valve 90. Specifically, the screw-coupling structure 52 forms a lower chamber 525, and the check valve 90A includes a valve body 91A, a fixing part 92A and a stopper 93. The valve body 91A is located in the lower chamber 525 and has a through hole 912. The stopper 93 and the block 2324 are integrally formed.

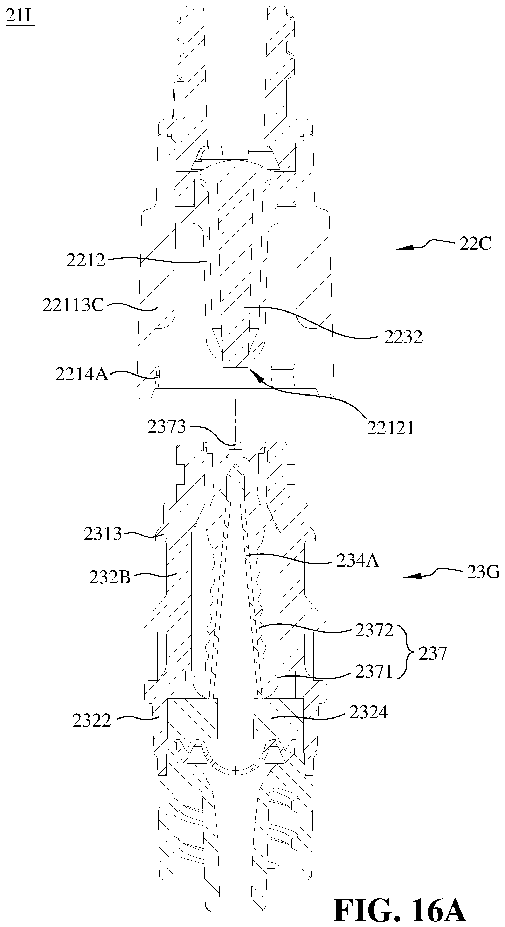

As shown in FIG. 16A, the structural difference between the movable closed connector 22C of the third closed needleless connector module 21I and the movable closed connector 22B of the third closed needleless connector module 21E is: the first engaging part 2214A may be disposed near the bottom of the lower tube part 22113C of the movable closed connector 22C. In other words, the first engaging part may be disposed at any position on the lower tube part of the movable closed connector.

As shown in FIG. 16A, the structural difference between the first needleless connector 23G of the third closed needleless connector module 21I and the first needleless connector 23F of the third closed needleless connector module 21H is that: first, the first needleless connector 23G includes a communication tube 234A and a fifth elastic valve 237. The communication tube 234A is provided in the second coupling part 232B and is conical in shape. Its top is a closed end and has a through hole (not shown). The through hole penetrates one side wall of the communication tube 234A and communicates with the inside of the communication tube 234A. The bottom end of the communication tube 234A is the block 2324, and the fifth elastic valve 237 is provided at the second coupling part 232B. The fifth elastic valve 237 has a fifth circumferential part 2371 and a fifth sealing part 2372.

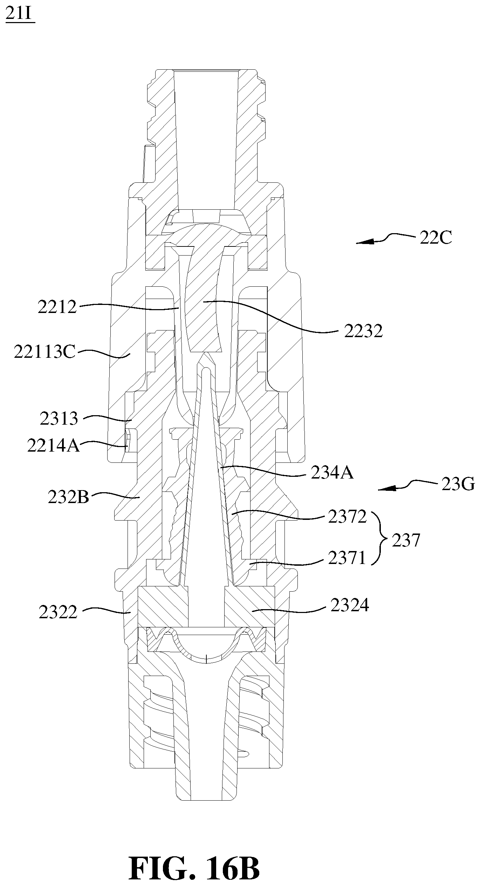

As shown in FIG. 16B, the difference in the docking methods between the third closed needleless connector module 21E and the third closed needleless connector modules 21F is that the communication tube 234A pushes to open a narrow slit 2373 of the fifth sealing part 2372 (see FIG. 16A), the communication tube 234A pushes against and compresses the first sealing part 2232, the communication tube 234A passes through the first opening 22122 and enters the inside of the conduit 2212, the conduit 2212 compresses the fifth sealing part 2372, and the fifth circumferential part 2371 will not deform.

It is worth noting that the communication tubes 234 of the first needleless connectors 23C to 23F are movable, and the communication tube 234A of the first needleless connector 23G is fixed. In other words, any form of movable closed connector 22A, 22B, and 22C is suitable for the movable or fixed communication tubes 234 and 234A of the first needleless connectors 23C to 23G.

Here, the term "medical device" is used to refer to any medical device commonly used in the medical field, except that it can be implemented in any different shape other than the connector disclosed here, and can be applied to related connector devices to connect or combine with other medical devices.

Although the present invention has been described with reference to the preferred embodiments thereof, it is apparent to those skilled in the art that a variety of modifications and changes may be made without departing from the scope of the present invention which is intended to be defined by the appended claims.

## Claims

1. An easy to disengage separator, comprising:
a valve body;
a screw-coupling structure, an internal thread at a first end of the screw-coupling structure being threaded with an external thread at a first end of the valve body and defining an input end of the separator, the screw-coupling structure having an inner conduit;
an output end, a first end of the output end being disposed on a second end of the valve body; and
a snap-on needleless connector module, comprising:
a movable closed connector, includes a main body, a first coupling part and a first elastic valve; the main body comprising a sleeve, a conduit, a positioning part and a first engaging part, the conduit being arranged inside the sleeve and comprising a first channel and a first opening, the positioning part being located inside the sleeve and provided on the outside of the conduit, the first engaging part being a bump and provided on the sleeve, and the first coupling part being provided on the sleeve; the first elastic valve comprising a first circumferential part and a first sealing part, two ends of the first circumferential part being respectively provided on the positioning part and the first coupling part, the first sealing part being located in the first channel, a first end of the first sealing part connected to an inner side of the first circumferential part and a plurality of through holes being provided, the through holes communicating with the first channel, and a second end of the first sealing part closes the first opening; and
a needleless connector, comprising a shell, a second coupling part and a second elastic valve; the shell having a second engaging part, the second engaging part being a bump and provided on an outer side of the shell or an outer side of the second coupling part, a first end of the shell having a second opening, and the second coupling part being provided on a second end of the shell, having a third opening, and being combined with the screw-coupling structure; and the third opening communicating with the inner conduit; the second elastic valve comprising a second circumferential part and a second sealing part; the second circumferential part being provided inside the shell and the second coupling part and having a second channel, and the second channel communicating with the third opening; the third opening communicating with the inner conduit.; the second sealing part being provided inside the shell and having a slit, and the second sealing part sealing the second opening;
wherein, when the movable closed connector is docked with the needleless connector, the first engaging part is fixed on the second engaging part, and the force of the first engaging part being fixed on the second engaging part is 1.4-8.1 Newton-meters, the conduit compresses the second sealing part, the second sealing part compresses the second circumferential part, so that the second sealing part is away from the second opening, and the conduit passes through the slit of the second sealing part and enter the inside of the second sealing part, the first end of the shell is against an inner wall of the sleeve, the positioning part moves along the conduit in a direction away from the needleless connector and compresses the first circumferential part, so that the second end of the first sealing part is separated from the first opening, and the first channel and the second channel become communicated with the inner conduit; and
wherein, when the movable closed connector is separated from the needleless connector, the first engaging part is disengaged from the second engaging part, and the conduit is separated from the second sealing part, so that the slit of the second sealing part becomes closely fitting, the second sealing part is restored by the elastic force of the second circumferential part and closes the second opening, the first end of the shell is separated from the sleeve, and the positioning part and the first sealing part are reset by the elastic reset of the first circumferential part to cause the second end of the first sealing part to seals the first opening.

2. The easy to disengage separator according to claim 1, wherein the sleeve comprises an upper tube part, a middle tube part and a lower tube part; the middle tube part is arranged inside a first end of the upper tube part; the lower tube part is arranged on the outer side of the first end of the upper tube part, so that the middle tube part extends inside the lower tube part;
wherein the conduit extends from the inside of the upper tube part through the inside of the middle tube part to the inside of the lower tube part;
wherein the positioning part is located inside the upper tube part;
wherein, the first engaging part is provided on an inner side of the lower tube part, and is located below the middle tube part;
wherein, the first coupling part is provided on a second end of the upper tube part;
wherein, the shell includes an upper shell and a lower shell, a first end of the upper shell has the second opening, an outer diameter of the upper shell is smaller than an outer diameter of the lower shell, and the second engaging part is provided on an outer side of a first end of the lower shell;
wherein, when the movable closed connector is docked with the needleless connector, the first engaging part is fixed to a bottom end of the second engaging part, a first end of the upper shell abuts against an inner wall of the middle tube part.

3. The easy to disengage separator according to claim 2, wherein when the height of the first engaging part protruding from the lower tube part is 0.05mm, the force of fixing the first engaging part to the second engaging part is 2.7 Newton-meters; wherein, when the height of the first engaging part protruding from the lower tube part is 0.07mm, the force of fixing the first engaging part to the second engaging part is 4.1 Newton-meters; wherein, when the height of the first engaging part protruding from the lower tube part is 0.1mm, the force of fixing the first engaging part to the second engaging part is 5.4 Newton-meters; wherein, when the height of the first engaging part protruding from the lower tube part is 0.14mm, the force of fixing the first engaging part to the second engaging part is 8.1 Newton-meters.

4. The easy to disengage separator according to claim 1, further comprising a balance pressure valve disposed between the second coupling part and the screw-coupling structure.

5. The easy to disengage separator according to claim 1, further comprising a check valve disposed between the second coupling part and the screw-coupling connection structure.

6. An easy to disengage separator, comprising:
a valve body;
a screw-coupling structure, an internal thread at a first end of the screw-coupling structure being threaded with an external thread at a first end of the valve body and defining an input end of the separator, the screw-coupling structure having an inner conduit;
an output end, a first end of the output end being disposed on a second end of the valve body; and
a snap-on needleless connector module, comprising:
a movable closed connector, includes a main body, a first coupling part and a first elastic valve; the main body comprising a sleeve, a conduit, a positioning part and a first engaging part, the conduit being arranged inside the sleeve and comprising a first channel and a first opening, the positioning part being located inside the sleeve and provided on the outside of the conduit, the first engaging part being a bump and provided on the sleeve, and the first coupling part being provided on the sleeve; the first elastic valve comprising a first circumferential part and a first sealing part, one end of the first circumferential part being provided on the positioning part, the first sealing part being located in the first channel, a first end of the first sealing part connected to an inner side of the first circumferential part and a plurality of through holes being provided, the through holes communicating with the first channel, and a second end of the first sealing part closes the first opening; and
a needleless connector, comprising a shell, a second coupling part, a communication tube, a third elastic valve, and a fourth elastic valve; the shell or the second coupling part having a second engaging part, and the second engaging part being a bump and being provided on an outer side of the shell or an outer side of the second coupling part; a first end of the shell having a second opening, and the second coupling part being provided on a second end of the shell, having a third opening, and being integrally formed with the screw-coupling structure; the third opening communicating with the inner conduit; the communication tube being provided in the shell and comprising an upper cylinder, a large-diameter part and a lower cylinder; the upper cylinder being arranged above the large-diameter part and having a closed top end, and being provided with at least one through hole; the at least one through hole penetrating one side wall of the upper cylinder and communicating with the inside of the upper cylinder' the lower cylinder being arranged below the large-diameter part and having an open bottom end; the inside of the upper cylinder, the inside of the large-diameter part, and the inside of the lower cylinder communicating with one another; the third elastic valve being arranged in the shell and comprising a third circumferential part and a third sealing part; the third circumferential part being arranged around the outside of the upper cylinder and its bottom end abutting against the large-diameter part, the third sealing part being provided at the top of the third circumferential part and having a slit; the fourth elastic valve being provided in the shell and comprising a fourth circumferential part and a fourth sealing part, the fourth circumferential part being arranged around the outside of the lower cylinder and its top end abutting against the large-diameter part, and the fourth sealing part being arranged at the bottom end of the fourth circumferential part and abutting against an inner wall of the second coupling part; a second end of the shell pressing the top of the fourth sealing part, the fourth sealing part having a slit, and the slit of the fourth sealing part being aligned with the inside of the lower cylinder and the third opening;
wherein, when the movable closed connector is docked with the needleless connector, the first engaging part is fixed on the second engaging part, and the force of the first engaging part being fixed on the second engaging part is 1.4-8.1 Newton-meters, the upper cylinder pushes open the slit of the third sealing part, the upper cylinder pushes against and compresses the first sealing part, and the upper cylinder passes through the first opening and enters the inside of the conduit, the conduit compresses the third sealing part, the third sealing part compresses the third circumferential part, so that the at least one through hole of the upper cylinder is exposed outside the third sealing part and is located inside the conduit, the third circumferential part pushes against the large-diameter part, the large-diameter part compresses the fourth circumferential part, and the lower cylinder pushes open the slit of the fourth sealing part and enters the third opening, so that the first channel, the communication tube, and the inner conduit are connected; and
wherein, when the movable closed connector is separated from the needleless connector, the first engaging part is disengaged from the second engaging part, and the fourth circumferential part is pushed against the large-diameter part by its elastic force, so that the communication tube and the third elastic valve move, and the lower cylinder returns to the inside of the fourth circumferential part to make the slit of the fourth sealing part tightly closed; the third circumferential part is pushed against the conduit by its elastic force to drive the movable closed connector to move, causing the upper cylinder to break away from the conduit and return to the inside of the third circumferential part; the conduit breaks away from the third sealing part, causing the slit of the third sealing part to be tightly closed.; the third sealing part is restored by the elastic force of the third circumferential part and closes the second opening, the first end of the shell is disengaged from the sleeve, and the first sealing part is reset by its elastic force, so that the second end of the first sealing part seals the first opening

7. The easy to disengage separator according to claim 6, wherein the sleeve comprises an upper tube part and a lower tube part; the lower tube part is arranged on the outer side of the first end of the upper tube part;
wherein the conduit extends from the inside of the upper tube part to the inside of the lower tube part;
wherein the positioning part is located inside the upper tube part;
wherein, the first engaging part is provided on an inner side of the lower tube part;
wherein, the first coupling part is provided on a second end of the upper tube part;
wherein, the shell includes an upper shell and a lower shell, a first end of the upper shell has the second opening, the lower shell presses the top end of the fourth sealing part, and the second engaging part is provided on an outer side of a first end of the lower shell;
wherein, when the movable sealing connector is docked with the needleless connector, the first engaging part is fixed to a bottom end of the second engaging part.

8. The easy to disengage separator according to claim 7, wherein when the height of the first engaging part protruding from the lower tube part is 0.05mm, the force of fixing the first engaging part to the second engaging part is 2.7 Newton-meters; wherein, when the height of the first engaging part protruding from the lower tube part is 0.07mm, the force of fixing the first engaging part to the second engaging part is 4.1 Newton-meters; wherein, when the height of the first engaging part protruding from the lower tube part is 0.1mm, the force of fixing the first engaging part to the second engaging part is 5.4 Newton-meters; wherein, when the height of the first engaging part protruding from the lower tube part is 0.14mm, the force of fixing the first engaging part to the second engaging part is 8.1 Newton-meters.

9. The easy to disengage separator according to claim 6, further comprising a balance pressure valve disposed between the second coupling part and the screw-coupling structure.

10. The easy to disengage separator according to claim 6, further comprising a check valve disposed between the second coupling part and the screw-coupling connection structure.
